# EUROPEAN PATENT SPECIFICATION

(11) **EP 0 656 064 B1**
(45) Date of publication and mention of the grant of the patent: **05.03.1997**
(21) Application number: 93920182.8
(22) Date of filing: 17.08.1993
(51) Int. Cl.: C12N 15/62, C12N 15/13, C12N 15/12, C07K 19/00, C07K 16/28, C07K 16/10

(54) **BISPECIFIC IMMUNOADHESINS**
BISPEZIFISCHE IMMUNOADHESINE
IMMUNOADHESINES BISPECIFIQUES

(30) Priority: 17.08.1992 US 931811
(43) Date of publication of application: 07.06.1995
(73) Proprietor: GENENTECH, INC., South San Francisco California 94080 (US)
(72) Inventor: ASHKENAZI, Avi, J., San Mateo, CA 94402 (US); CHAMOW, Steven, M., San Mateo, CA 94403 (US)
(74) Representative: Greaves, Carol Pauline
(86) International application number: US9307783
(87) International publication number: WO9404690

(56) References cited:
- PROCEEDINGS OF THE NATIONAL ACADEMY OF SCIENCES OF USA. vol. 88, no. 11 , 1 June 1991 , WASHINGTON US pages 4723 - 4727 J.BERG ET AL. 'Bispecific antibodies that mediate killing of cells infected with human immunodeficiency virus of any strain' cited in the application
- EMBO JOURNAL. vol. 10, no. 12 , December 1991 , EYNSHAM, OXFORD GB pages 3655 - 3659 A.TRAUNECKER ET AL. 'Bispecific single chain molecules (Janusins) target cytotoxic lymphocytes on HIV infected cells' cited in the application
- NATURE. vol. 344 , 12 April 1990 , LONDON GB pages 667 - 670 R.A.BYRN ET AL. 'Biological properties of a CD4 immunoadhesin' cited in the application

## Description

### Field of the Invention

The present invention is from the field of bispecific immunoadhesins. More specifically, this invention relates to the use of recombinant techniques to produce immunoadhesins with dual specificity and to techniques for their purification to homogeneity. The invention also concerns bispecific immunoadhesins for therapeutic or diagnostic use.

### Background of the Invention

Immunoadhesins are antibody-like molecules which combine the binding specificity of a protein such as a cell-surface receptor, a cell-adhesion molecule or a ligand (an "adhesin"), with the effector functions of immunoglobulin constant domains. Immunoadhesins can possess many of the valuable chemical and biological properties of human antibodies. Since immunoadhesins can be constructed from a human protein sequence with a desired specificity linked to an appropriate human immunoglobulin hinge and constant domain (Fc) sequence, the binding specificity of interest can be achieved using entirely human components. Such immunoadhesins are minimally immunogenic to the patient, and are safe for chronic or repeated use. If the two arms of the antibody-like immunoadhesin structure have two different specificities, the immunoadhesin is referred to as bispecific on the analogy of bispecific antibodies.

Immunoadhesins reported in the literature include fusions of the T cell receptor* [Gascoigne *et al.,* Proc. Natl.Acad. Sci. USA 84, 2936-2940 (1987)]; CD4* [Capon *et al.,* Nature 337, 525-531 (1989); Traunecker *et al.*, Nature 339, 68-70 (1989); Zettmeissl *et al.,* DNA Cell Biol. USA 9, 347-353 (1990); Byrn *et al.,* Nature 344, 667-670 (1990)]; L-selectin (homing receptor) [Watson *et al.*, J. Cell. Biol. 110, 2221-2229 (1990); Watson *et al.,* Nature 349, 164-167 (1991)]; CD44* [Aruffo *et al.,* Cell 61, 1303-1313 (1990)]; CD28* and B7* [Linsley *et al.,* J. Exp. Med. 173, 721-730 (1991)]; CTLA-4* [Lisley *et al.*, J. Exp. Med. 174, 561-569 (1991)]; CD22* [Stamenkovic *et al*., Cell 66. 1133-1144 (1991)]; TNF receptor [Ashkenazi *et al*., Proc. Natl. Acad. Sci. USA 88, 10535-10539 (1991); Lesslauer *et al.,* Eur. J. Immunol. 27, 2883-2886 (1991); Peppel *et al.,* J. Exp. Med. 174, 1483-1489 (1991)]; NP receptors [Bennett *et al.,* J. Biol. Chem. 266, 23060-23067 (1991)]; IgE receptor α-chain* [Ridgway and Gorman, J. Cell. Biol. 115, abstr. 1448 (1991)]; HGF receptor [Mark, M.R. *et al.*, 1992, J.Biol. Chem. 267, 26166-26171, where the asterisk (*) indicates that the receptor is member of the immunoglobulin superfamily.

A prototype of receptor-immunoglobulin immunoadhesins is the CD4-IgG chimera, which has been extensively studied in clinical trials to investigate its ability to prevent or treat human immunodeficiency virus (HIV) infections.

HIV depletes CD4⁺ lymphocytes by mechanisms that are still controversial and destroys the monitoring functions of the immune system in spite of the presence of antibodies to HIV antigens and cellular immune responders [Groopman *et al.,* AIDS Res. and Human Retrovirus 3, 71-85 (1987); Walker *et al.,* Nature 328, 345-348 (1986)].

Cytotoxic cells fall into three main categories; mediators of antibody-dependent cellular cytotoxicity (ADCC), cytotoxic T lymphocytes (CTL), and promiscuous killer cells, including natural killer (NK) cells and activated macrophages [Henkart, P.A., Ann. Rev. Immunol. 3:31-58 (1985)]. In T cell-mediated lysis, the T cell receptor (TCR) binds to altered or foreign markers in association with class II major histocompatibility complex (MHC) molecules on tumor or infected cell surfaces to distinguish these cells from normal autologous cells. In ADCC, this function is carried out by Fcγ receptor (FCγR) to destroy the antibody coated target cell [Lovchik, J.C. and Hong, R., Prog. Allergy 22, 1-44 (1977); Zuckerman, S.H. and Douglas, S.D., CRC Crit. Rev. Microbiol. 7:1-26 (1978); and Unkeless, J.C., *et al.,* Adv. Immunol. 31:247-468 (1981)]. The normal immune responses to a foreign antigen include antigen processing and presenting by macrophages, monocytes and dendritic cells, T helper and memory cell regulation and the activation of these effector cells. CD4+ T helper cells play a central role in the normal cascade of immune system responses by producing lymphokines to stimulate B cell, evoking the humoral immunity response leading to ADCC, and by stimulating activation of cytotoxic T lymphocytes, thereby recruiting the cellular immune response to foreign pathogens. The latter is thought to be a major host defense against virus infection [Yap, K.L., *et al.*, Nature 273:238-239 (1978); Kannagi, M. *et al.*, J. Immunol. 130:2942-2946 (1983); Flyer, D.C., *et al.,* Nature 305:815-818 (1983); Holt, C.A., *et al.*, J. Exp. Med. 164:211-226 (1986)]. This sequence is broken in HIV infection: antigen-presenting cells are infected and their functions decreased. CD4⁺ T helper cells are progressively depleted and their activities are diminished with concomitant loss of "help" to both cellular and humoral system and consequent failure of the immune system to control the virus infection.

In 1984, the cell surface protein CD4 was shown to be the receptor for the human immunodeficiency virus, HIV [reviewed in Capon and Ward, Annu. Rev. Immunol. 9, 649-678 (1991)]. As the hallmark of HIV infection is the specific destruction of the CD4⁺ T cells, and the progression of infected individuals to AIDS closely parallels their decline in CD4⁺ T-cell number [Lane, H. and Fauci, A., Annu. Rev. Immunol. 3, 477-500 (1985)], it was proposed that the interaction of the HIV-1 envelope glycoprotein, gp120 with the CD4 receptor, either by direct HIV infection of CD4⁺ cells or otherwise, underlies the killing of CD4⁺ cells.

Truncation of the native CD4 gene to remove sequences which encode the transmembrane and cytoplasmic domains enables the production of a recombinant soluble, secreted protein. It was suggested by several groups that recombinant soluble CD4 (rsCD4) might prevent HIV infection by blocking the virus-receptor interaction. However, it soon became clear that, due to their small size, the half-lives of soluble, secreted forms of the CD4 antigen were short as molecules of this size (about 50 kDa) are swiftly cleared by the kidney. In view of their structural similarity, it appeared possible that part of CD4 could be substituted for the variable regions of immunoglobulins without adversely effecting either portion of the chimeric molecule, and it was suggested that this molecule would have significantly altered pharmacokinetics with a longer half-life and slower clearance, producing much higher steady state concentrations for equivalent doses. As such constructions contain the Fc domain of an immunoglobulin, it was predicted that they would also acquire the defensive properties of an antibody molecule, such as the ability to mediate antibody-dependent cellular cytotoxicity (ADCC), activation of the lytic complement cascade, and improved removal of virus due to Fc receptor-mediated clearance.

Immunoadhesins combining different portions of CD4 with different portions of immunoglobulins are disclosed in EP 314,317 (published 3 May 1989). A pharmacokinetic study in rabbits indicated about a 30-fold reduction in the rate of clearance of the immunoadhesin designated CD4₂Fcl, as compared to that of recombinant soluble CD4 (rsCD4). A similar difference in the clearance rate was observed in monkeys and in humans [Mordenti *et al.,* Pharmaceut. Res. 8, 1351-1359 (1991)]. Others [Zeittemeissl *et al.,* DNA Cell Biol. (US) 9, 347-353 (1990)] have also observed a dramatically slower clearance of similar CD4-Ig immunoadhesins in mice and monkeys.

Byrn *et al.,* Nature 344, 667-670 (1990) showed that CD4- IgG can mediate ADCC of HIV-infected cells to the same extent as polyclonal anti-HIV sera. Importantly, however, while anti-HIV antisera can also mediate ADCC on "bystander" cells sensitized by the presence of soluble gp120, this is not the case for CD4-IgG, since soluble gp120 has only one CD4-binding site. Byrn *et al.,* *supra* also showed that CD4-IgG was transferred across the primate placenta with a rate of 1% per 8 hours, comparable to that observed for human IgG in human subjects. This has now been confirmed in Phase I clinical trials in which 1 mg/kg i.v. bolus dose of CD4-IgG was administered to HIV seropositive pregnant women at the onset of labor or one week prior to delivery and at the onset of labor. CD4-IgG crossed the human placenta to the same extent as IgG does. Neither the mothers nor their infants showed any sign of toxicity. During the follow-up time none of the infants has shown evidence of HIV infection, as determined through PCR, plasma viral cultures, and lymphocyte proliferation.

It is known that although AIDS patients lose HIV-specific cytotoxic T cells, their remaining CD8-positive T lymphocytes maintain cytotoxic function. It has also been shown that cytotoxic T cells of irrelevant specificity can be focused onto and stimulated to kill target cells by means of bispecific antibodies [see Staerz, U.D. and Bevan, M.J., Immunol. Today 7, 241-245 (1986) and the references cited therein]. Cytotoxic T lymphocytes may be the last survivors of the disasterous failure of the immune system due to HIV infection, with their function retained [Walker, B.D., Nature 328:345-348 (1986)] although not activated or targeted to virus. Therefore, a rational approach to recruiting the immune system may be to exploit this remaining cellular immunity function through retargeting in the combat with virus.

In an attempt to avoid the blocking of the immune system activation pathway in AIDS patients while retaining the anti-retroviral properties of CD4-immunoglobulin chimeras, bispecific antibodies capable of retargeting effector cells against virus infected cells were made. Because they express integral viral proteins on the surface, cells infected by retroviruses have been proposed to be particularly suitable for the bispecific antibody approach. With one arm recognizing a foreign antigen on the surface of a target cell and with the other arm recognizing an activation molecule on the surface of cytotoxic cells, such antibody-like molecules are able to activate the effector cells through their signal transduction systems, and help to deliver a lethal hit to the target cell even without the participation of the MHC system [Segal, D and Wunderlich, J., Cancer Invest. 6, 83-92 (1988); Junghans, R.P., Ann. Inter. Med. 116, 146-160 (1992)].

With this new generation of antibody-like molecules, often termed bifunctional antibody in the literature, it is possible to avoid the functional defects of antigen-presenting, and immunoregulatory cells can activate the effector cells directly to respond to HIV-1 infection. This is mediated by binding of these artificial antibodies to foreign antigens on the surface of infected cells and to Fc receptors on the ADCC mediator cell or to antigen receptor on the CTLs.

In an attempt to provide an improved immunotherapy for AIDS, Berg *et al.,* Proc. Natl. Acad. Sci. USA 88, 4723 (1991) constructed a bispecific antibody-like molecule (bispecific immunoadhesin with the terminology used herein) comprising a heavy chain/light chain pair from a mouse antibody to human CD3, linked to a human IgG heavy chain whose variable region has been replaced with a CD4 sequence, plus a second light chain. CD3 is part of the antigen receptor on T cells and is responsible for signal transduction. The bispecific molecule was designed to combine the ability of CD4 to bind to the gp120 of any HIV strain with the T-cell activation property of antibodies to CD3.

At present, the recombinant production of bispecific antibodies and immunoadhesins is usually based on the coexpression of two immunoglobulin heavy chain-light chain pairs, where the two heavy chains have different specificities [Milstein and Cuello, Nature 305, 537-539 (1983)]. The presence of the light chains is generally thought to be necessary for efficient secretion, since immunoglobulin heavy chains not linked to light chains had been shown to be held back by an immunoglobulin heavy chain binding protein (BiP) in the endoplasmic reticulum [Haas, I.G. Wabl, M.R., Nature 306, 387-389 (1983); Bole, D.G. *et al.*, J. Cell Biol. 102, 1558-1566 (1986)]. Because of the random assortment of immunoglobulin heavy and light chains, these hybridomas (quadromas) produce a potential mixture of 10 different antibody molecules, of which only one has the correct bispecific structure. The purification of the correct molecule, therefore, is very cumbersome. Thus, because only one of 10 possible structures is the desired one, and because the purificaton of that molecule is difficult, the yield of the desired product is decreased considerably.

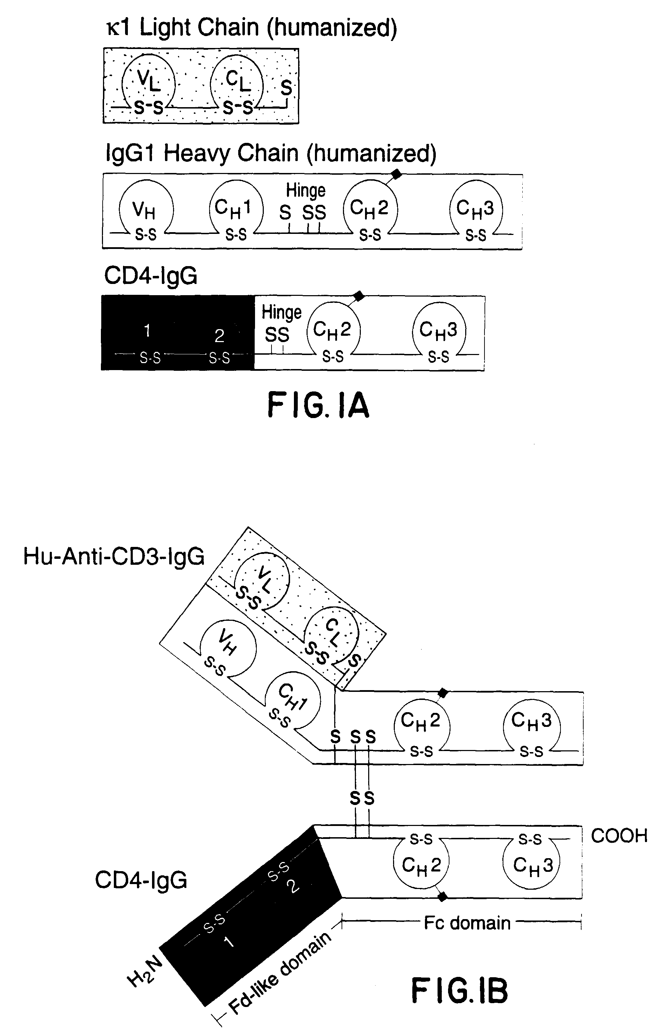

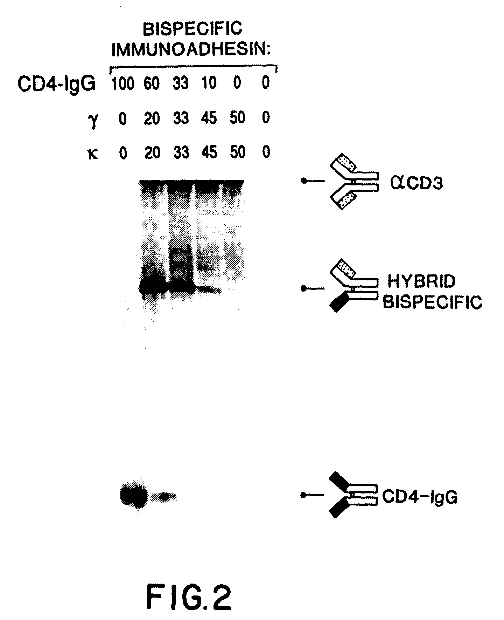

The mouse bispecific immunoadhesin of Berg *et al.* was made on the analogy of bispecific antibodies, by cloning the gene encoding the chimeric protein CD4₂γ1 (Capon *et al.,* supra) into a vector that allows expression in mouse cells and transfecting the vector obtained into a hybridoma secreting a γ1, anti-human CD3 antibody. As CD4₂γ1 retains the CH1 domain of an immunoglobulin, it has the ability to bind an antibody light chain. Accordingly, the transfectoma produced a heterogeneous product mixture, including the desired product which is a tetramer consisting of CD4₂γ1 disulfide-bonded to an anti-CD3 antibody light chain linked to an anti-CD3 antibody heavy chain-light chain pair (Figure 1 of Berg *et al.*). Other combinations of the three polypeptide chains present in the transfectoma included two bivalent monospecific tetramers consisting of two disulfide-linked CD4₂γ1-anti-CD3 antibody light chain pairs (bivalent monospecific immunoadhesin), and two disulfide-linked anti-CD3 antibody heavy chain-light chain pairs (bivalent anti-CD3 antibody), respectively. The CD4-region containing products were separated from the bivalent anti-CD3 antibodies by anti-CD4 affinity chromatography using an unidentified anti-CD4 monoclonal antibody for elution. As attested by the electrophorograms in Figure 2E of Berg *et al.,* roughly equal portions of the desired bifunctional immunoadhesin, and a tetramer of two bivalent monofunctional disulfide-linked CD4₂γ1-anti-CD3 antibody light chain pairs were obtained. These two components were not separated, and the bispecific immunoadhesin preparations tested for efficacy in promoting the killing of HIV-infected cells *in vitro* contained an equal amount of monospecific bivalent CD4₂γ1, tetramers (which do not promote cell killing) and the desired, active bispecific immunoadhesin. The difficulty of purification lies in the small quantities and in the structural similarity of the two components, both having antibody-like, tetrameric structures consisting of two disulfide-bonded heavy chain-light chain pairs. The separation of these two structurally very similar products would have to be based on the CD3 binding site, and would have required reagents, such as purified CD3 ligand or an anti-idiotypic monoclonal antibody for the CD3 binding site, that are not readily available in preparation quantities.

Traunecker *et al.*, EMBO 10, 3655-3659 (1991) generated two kinds of bispecific CD4-anti-CD3 molecules. The first structure was based on human IgG3 whose Fab regions were replaced with anti-CD3 single chain combining site (FvCD3) and the first two N-terminal domains of CD4. F i r s t , variable domain sequences from anti-CD3 hybridoma TR66 (V_{H} and V_{L}) were cloned and fused together with a synthetic piece of DNA encoding an 18 amino acid linking peptide between the V_{H} and V_{L} domains, to yield a structure designated FvCD3. FvCD3 was then used to replace the variable domains in a human IgG3 molecule, and the obtained immunoadhesin (FvCD3-Hγ3) was coexpressed with CD4-Hγ3 in mammalian cells. Three major products (CD4-Hγ3, FvCD3-Hγ3, and CD4-Hγ3/FvCD3-Hγ3) were obtained, of which the last mentioned bispecific immunoadhesin was a minor component which was not purified.

To overcome the difficulties experienced with the bispecific immunoadhesin structure, Traunecker *et al.* designed a single-chain polypeptide, containing the FvCD3, the two N-terminal domains of CD4 and C-kappa, designated Janusin (CD4-FvCD3-Ckappa). This bispecific linear molecule was purified by using anti-kappa affinity columns. As this molecule contains no immunoglobulin constant domain sequences, its plasma half-life is not expected to be significantly longer than that of soluble CD4.

In summary, the coupling of a gp120-interaction domain in the form of CD4 [Berg, J. *et al.,* Supra; Traunecker, A., *et al.,* Supra] or an anti-gp120 monoclonal antibody [Zarling, J. M. *et al.,* Supra; Okada, H. *et al.,* Supra] to bind to HIV antigen, with a CD3-interaction domain to stimulate cytotoxic T lymphocytes, has so far resulted in immunoglobulin-like molecules [Berg, J. *et al.,* Supra; Zarling, J. M. *et al.,* J. Immunol. 140, 2609-2613 (1988); Okada, H. *et al.,* Immunol. Lett. 15, 31.247-52 (1992)] or a single peptide chain [Traunecker, A., *et al.,* Supra]. Preparations containing such bispecific constructs were effective at inducing lysis of HIV-infected cells with cytotoxic T lymphocytes. Other approaches [Zarling, J. M. *et al.,* Supra] also exploited an anti-CD16 (3G8) specificity to recruit killing by large granular lymphocytes (LGLs), a principal mediator of ADCC against nucleated cell targets *in vivo* [Ortaldo, J.R., *et al.,* J. Immunol. 138: 3566-3572 (1987)]. This showed cytotoxic potency equivalent to that seen with an anti-CD3 bispecific constructs recruiting CTL killing in the same system.

Despite the recent advances made in this area, at present there is no generally applicable method known in the art for the efficient production, isolation and purification of bispecific immunoadhesins. The yield of the desired product is usually low especially when the number of possible chain combinations is large, and the separation of the bispecific immunoadhesin molecule from by-products with unwanted chain combinations requires the availability of specific separation methods, e.g. specifically binding reagents for each of the functionalities. When there is no easy way to separate the components of the product mixture based on the two specificities, the separation and purification become cumbersome and prohibitively expensive and time consuming.

It would be desirable to provide an efficient method for the high-yield production of bispecific immunoadhesins with any desired specificities.

It would also be desirable to provide a method that enables the separation of a bispecific immunoadhesin from accompanying by-products resulting from unwanted immunoglobulin chain combinations even when there is no readily available reagent specifically recognizing one of the specificities, so that the desired bispecific product can be isolated in substantially homogeneous form.

It would further be desirable to provide a method for the prevention of treatment of HIV infection in an individual exposed to HIV by retargeting the patient's own cytotoxic lymphocytes to kill HIV-infected cells.

Accordingly, it is an object of the present invention to provide a method for the production of substantially homogeneous bispecific immunoadhesins.

It is another object, to provide a method for the separation of a bispecific immunoadhesins from accompanying by-products in substantially homogeneous form.

It is a further object, to provide substantially homogeneous bispecific immunoadhesins obtainable by such methods.

It is yet another object to provide a method for the prevention or treatment of HIV infection in individuals that have been exposed to or are at a risk to be exposed to HIV, including the prevention of the transmission of HIV infection from infected pregnant women to the fetus.

It is a further object to provide a drug delivery system capable of specifically targeting an anti-HIV agent to the cytotoxic lymphocytes or to the lymph nodes of patients having been exposed to or infected by HIV.

These and further objects will be apparent for one skilled in the art.

### Summary of the Invention

The invention relates to a novel method for making and purifying bispecific immunoadhesins.

Based upon research with antibodies, it is generally believed that the presence of light chain is essential for efficient secretion of immunoadhesins (see, e.g. Berg *et al.,* supra). Most bispecific immunoadhesins known in the art have been designed to have a light chain associated with a heavy chain fusion protein in each arm of the molecule. In the rare instances when the light chain is absent (see U.S. patent No. 5,116,964 issued 26 May 1992) the bispecific immunoadhesin has a symmetrical structure to ensure proper assembly and folding of the hybrid immunoglobulin chains.

The present invention takes advantage of the experimental finding that bispecific immunoadhesins composed of a hybrid immunoglobulin heavy chain in one arm and a hybrid immunoglobulin heavy chain-light chain pair in the other arm can be efficiently secreted in the form of correctly assembled and folded heterotrimers. This is notwithstanding the absence of the light chain in one arm, and the asymmetric structure of the trimeric molecule. It was further found that the same asymmetric structure that is not a detriment in the preparation process, facilitates the separation of the desired bispecific compound from unwanted immunoglobulin chain combinations, as the presence of an immunoglobulin light chain in only one half of the bispecific molecule provides for a facile way of separation.

The bispecific immunoadhesins of the invention are preferably produced by individually introducing into suitable host cells the DNA sequences encoding the three immunoglobulin chains making up the trimeric molecule. As a result, the ratios of these DNA sequences can be freely changed. Utilizing the flexibility of this system, it has been found that the ratio in which the DNA sequences encoding the three chains of a heterotrimeric bispecific immunoadhesin are introduced into a recombinant host cell has a genuine effect on the composition of the product mixture obtained by their expression. More specifically, the mutual proportions of the three possible immunoglobulin chain combinations have been found to vary significantly as a function of the ratio of the plasmid DNAs used for transfection. Accordingly, by proper adjustment of the ratios of the DNA sequences encoding the two individual hybrid immunoglobulin heavy chains and the light chain the yield of any desired bispecific immunoadhesin product can be optimized.

For the immunoadhesins with CD4 and anti-CD3 specificities it has been found that by using an excess amount of the DNA encoding the CD4-immunoglobulin fusion protein, the yield of the desired bispecific immunoadhesin molecule was markedly increased.

The advantages of the preparation and purification processes of the present invention over the traditional approach are best illustrated when compared to the purification scheme of the construct of Berg *et al.,* supra. The bispecific immunoadhesin of Berg *et al.* is composed of a mouse anti-human CD3 antibody heavy chain-light chain pair, disulfide linked to a CD4-IgG-1 heavy chain constant domain-mouse anti-human CD3 antibody light chain pair. In this design both arms of the bispecific antibody-like molecule comprise an antibody light chain disulfide linked to an immunoglobulin CH1 domain. The coexpression results in three major products: the bispecific immunoadhesin, a bivalent monospecific tetramer composed of two disulfide linked anti-CD3-antibody heavy chain-light chain pairs, and a second bivalent monospecific tetramer composed of two disulfide linked CD4-IgG-1 heavy chain-anti-CD3 light chain pairs. Whereas the CD4 sequence-containing products can be separated from the anti-CD3 antibody molecule by purification techniques based on the CD4 portion of the molecules, their separation from one another has not been accomplished, primarily due to the unavailability of reagents capable of selective binding to the anti-CD3 portion of the bispecific immunoadhesin molecule. As a result, Berg *et al.* end up with a product mixture not suitable for any commercial (therapeutic or diagnostic) application.

In contrast, the bispecific immunoadhesin structure of the present invention is composed of a humanized anti-CD3 antibody heavy chain-light chain pair, disulfide linked to a single CD4-IgG-1 heavy chain constant domain fusion protein from which the CH1 domain has been eliminated to avoid the binding of a light chain to this arm of the molecule. Moreover, instead of the transfectoma method commonly used for the preparation of full-length bispecific antibodies, the coding sequences of the three chains present in this structure were individually introduced into an appropriate host cell, thus providing for greater flexibility in adjusting their mutual proportions, and consequently resulting in markedly higher product yields than previously achieved. This technique resulted in three products: a heterotetramer composed of two immunoglobulin heavy chain-light chain pairs containing two binding sites for CD3; a homodimer composed of two CD4-Ig immunoadhesins containing two binding sites for gp 120 without associated light chains; and a heterotrimer composed of one immunoglobulin light chain, one immunoglobulin heavy chain and one CD4-Ig immunoadhesin, which contains one binding site for CD3 and one for gp 120 (bispecific immunoadhesin) . With a straightforward purification scheme, based on the CD4 portion and on the immunoglobulin light chain present in the bispecific molecule, the bispecific immunoadhesin can be separated from the undesired chain combinations in substantially homogenous form, enabling its therapeutic use.

Accordingly, the present invention relates to a method of making a bispecific immunoadhesin comprising:
a) introducing into a host cell DNA sequences encoding a first fusion comprising a first binding domain fused to an immunoglobulin heavy chain constant domain sequence lacking a light chain binding site; a second fusion comprising a second binding domain fused to an immunoglobulin heavy chain constant domain sequence retaining a light chain binding site; and an immunoglobulin light chain, respectively;
b) culturing the host cells so as to express the DNA sequences to produce a mixture of (i) a heterotrimer comprising the first fusion covalently linked with a second fusion-immunoglobulin light chain pair; (ii) a heterotetramer comprising two covalently-linked second fusion-immunoglobulin light chain pairs; and (iii) a homodimer comprising two covalently-linked molecules of the first fusion;
c) removing the mixture of products (i), (ii) and (iii) from the cell culture; and
d) isolating product (i) in substantially homogenous form by separation methods based on the first binding domain and on the immunoglobulin light chain, respectively.

In a preferred embodiment, at least two of the immunoglobulin chain-encoding DNA sequences are introduced into the host cells individually, and the yield of the desired heterotrimer is optimized by adjusting the ratios of these DNA sequences.

In another aspect, the invention concerns a method for isolating a bispecific immunoadhesin from the foregoing product mixture by utilizing methods specifically recognizing and separating, in optional order, the first binding domain- and the immunoglobulin light chain-containing products.

The preferred method for isolation is chromatography, and in particular, immunoaffinity chromatography, based on the first binding domain (i.e. the CD4 portion), and on the immunoglobulin light chain portion of the bispecific immunoadhesin molecule.

In a further aspect, the invention relates to a method for making a substantially homogeneous bispecific immunoadhesin with one arm specifically recognizing a foreign antigen on a target cell, and with the other arm an activation molecule on a cytotoxic cell, comprising:
a) introducing into host cells DNA sequences encoding a fusion of a foreign antigen binding domain with an immunoglobulin heavy chain constant domain sequence in which the first immunoglobulin heavy chain constant domain (CH1) has been removed or modified such that it is no longer capable of binding an immunoglobulin light chain, a heavy chain of an antibody to an activation site on a cytotoxic cell, retaining a CH1 domain capable of light chain binding, and a light chain of said antibody;
b) culturing said host cells so as to express said DNA sequences to produce a mixture of (i) a heterotrimer bispecific immunoadhesin comprising a foreign antigen binding domain-immunoglobulin heavy chain fusion disulfide linked to a disulfide bonded antibody heavy chain-light chain pair; (ii) a disulfide-linked heterotetramer comprising two antibody heavy chain-light chain pairs; and (iii) a homodimer comprising two disulfide-linked foreign antigen binding domain-immunoglobulin heavy chain constant domain fusion;
c) removing the mixture of products (i), (ii) and (iii) from the cell culture;
d) separating the bispecific immunoadhesin (i) from products (ii) and (iii) by affinity chromatography, in optional order, based on the foreign antigen binding portion and on the light chain portion of the bispecific immunoadhesin molecule; and
e) recovering the bispecific immunoadhesin (i) in substantially homogeneous form.

The foreign antigen may, for example, be an integral viral protein of a retrovirus such as HIV, when the specific binding is to a site within the gp 160, gp 120 or gp 41 domain. A molecule known to bind gp 120 is the cell surface glycoprotein CD4. The cytotoxic cells to be activated preferably are cytotoxic T cells or large granular lymphocytes, and the activation molecule preferably is CD3 or CD16.

In a still further aspect, the invention concerns a method for isolating a substantially homogeneous heterotrimeric bispecific immunoadhesin composed of an immunoglobulin heavy chain with a first functionality covalently linked with an immunoglobulin heavy chain-light chain pair with a second functionality from a mixture with a homodimer composed of two covalently linked immunoglobulin heavy chains of the first functionality and a heterotetramer composed of two covalently linked heavy chain light chain pairs of the second functionality, which comprises separating the heterotrimeric bispecific immunoadhesin from the accompanying heterotetramer and homodimer by two-step chromatography, in optional order, based on the first functionality and on the immunoglobulin light chain, respectively. If desired, at least one of the chromatographic purification steps may be repeated.

In yet another aspect, the invention relates to a substantially homogeneous bispecific immunoadhesin comprising a binding domain for an integral viral protein of a retrovirus fused to an immunoglobulin heavy chain constant domain sequence lacking an immunoglobulin light chain binding site covalently linked to a heavy chain-light chain pair of an antibody to an activation molecule on the surface of a cytotoxic cell. In a particular embodiment, the bispecific immunoadhesin is designed to have an HIV binding sequence and a sequence serving to retarget cytotoxic T lymphocytes or large granular lymphocytes to fight HIV infection.

In a further aspect, the invention concerns a method for the prevention or treatment of HIV infection in a human exposed to HIV, by administering an effective amount of a trimeric bispecific immunoadhesin with one arm specifically recognizing HIV on a target cell, and with the other arm an activation molecule on a cytotoxic cell.

In a still further aspect, the invention concerns a method for preventing the transmission of HIV infection from an HIV seropositive pregnant women to the fetus by administering to the pregnant women an effective amount of a trimeric bispecific IgG immunoadhesin with one arm specifically recognizing HIV on a target cell, and with the other arm an activation molecule on a cytotoxic cell.

Although the advantages of the preparation and purification methods herein have been illustrated by the example of a CD4-Ig-anti-CD3 antibody bispecific immunoadhesin [(CD4 x anti-CD3) bispecific immunoadhesin], it will be understood that the same scheme is broadly applicable to the preparation and purification to homogeneity of trimeric bispecific immunoadhesins irrespective of their specificities. Other bispecific immunoadhesins that can be made and purified in an analogous manner include, for example, CD4-IgG x anti-CD16 mAb, t-PA-IgG x anti-fibrin mAb, tumor necrosis factor receptor (TNFR)-IgG x anti-endotoxin mAb, CD4-IgG x TNFR-IgG, CD4-IgG x L-selectin-IgG etc. The last mentioned molecule combines the lymph node binding function of the lymphocyte homing receptor (LHR, L-selectin), and the HIV binding function of CD4, and finds potential application in the prevention or treatment of HIV infection, related conditions, or as a diagnostic.

All bispecific immunoadhesins of the present invention may be incorporated in liposomes, preferably in combination with other therapeutics with similar indications, thereby facilitating combination therapy.

### Brief Description of the Drawings

Figure 1 illustrates the structure of (CD4xanti-CD3) bispecific immunoadhesin. A. Subunit structures of the antibody heavy and light chains and the CD4-IgG-1 chimeric protein used in the construction of the bispecific immunoadhesin. B. Schematic structure of a (CD4xanti-CD3) bispecific immunoadhesin heterotrimer.

Figure 2 shows the production of the (CD4xanti-CD3) bispecific immunoadhesin heterotrimers, anti-CD3 (αCD3) heterotetramers, and CD4-immunoadhesin homodimers (CD4-IgG) using different proportions (drawn as percentage) of each DNA. The total amount of plasmid DNA was 20 µg in all cases. Transfected cells were metabolically labeled with ³⁵S-methionine, and the proteins were precipitated from serum-free supernatants using protein A and analysed by SDS-PAGE and autoradiography.

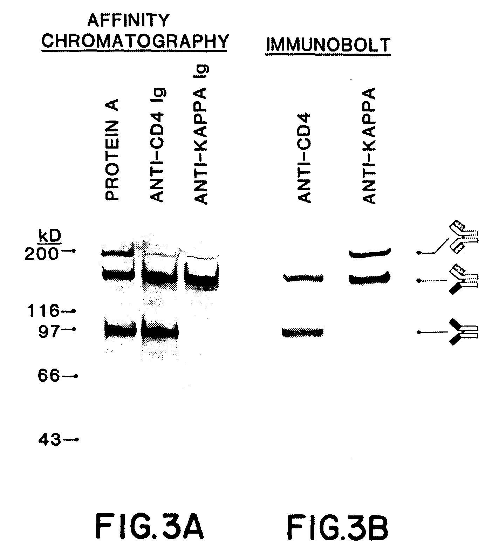

Figure 3 shows the separation of the (CD4xanti-CD3) bispecific immunoadhesin tererotrimer, the anti-CD3 heterotetramer, and the CD4-immunoadhesin homodimers by affinity chromatography as described in Example 1.

Figure 4. Specificity of lysis of uninfected cells (CEM) (a) and HIV infected cells (CEM/IIIB) (b) by effector cells plus antibody. Target cells were labeled overnight and incubated at room temperature with different antibodies at 1 µg/ml fr one hour, and then incubated at 37°C with the three effector cells, PBL-IL2 (black bars), PBL+IL-2 (white bars) and CTL (shaded bars), for three hours. Supernatants were harvested, counted and calculated as described in the examples.

Figure 5. Lysis of HIV infected cells (CEM/IIIB) by effector cells plus antibody in the absence (a) or in the presence of 8% (b) or 50% (c) human serum.

Figure 6. Dose dependent effect of bispecific immunoadhesin mediated CEM/IIIB lysis by CTL (the vertical bars show the standard deviations).

### Detailed Description of the Invention

### A. Definitions

Antibodies (Abs) and immunoglobulins (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas. In structural sense the terms "antibody" and "immunoglobulin" are used interchangeably throughout the specification, however, a clear distinction will be made when discussing functional features associated with antigen specificity.

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain chain also has regularly spaced intrachain disulfide bridges. Each heavy chain has at one end a variable domain (V_{H}) followed by a number of constant domains. Each light chain has a variable domain at one and (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains [Clothia *et al.,* J. Mol. Biol. 186, 651-663 (1985); Novotny and Haber, Proc. Natl. Acad. Sci. USA 82, 4592-4596 (1985)].

The variability is not evenly distributed through the variable regions of antibodies. It is concentrated in three segments called complementarity determining regions (CDRs) or hypervariable regions both in the light chain and the heavy chain variable regions. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies [see Kabat, E.A. *et al.,* Sequences of Proteins of Immunological Interest National Institute of Health, Bethesda, MD (1987)]. The constant domains are not involved directly in binding an antibody to an antigen, but exhibit various effector functions, such as participation of the antibody in antibody-dependent cellular toxicity.

Papain digestion of antibodies produces two identical antigen binding fragments, called Fab fragments, each with a single antigen binding site, and a residual "Fc" fragment, whose name reflects its ability to crystallize readily. Pepsin treatment yields an F(ab')₂ fragment that has two antigen combining sites and is still capable of cross-linking antigen.

"Fv" is the minimum antibody fragment which contains a complete antigen recognition and binding site. This region consists of a dimer of one heavy and one light chain variable domain in tight, non-covalent association. It is in this configuration that the three CDRs of each variable domain interact to define an antigen binding site on the surface of the V_{H}-V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

The light chains of immunoglobulins from any vertebrate species can be assigned to one of two clearly distinct types, called kappa and lambda (X), based on the amino acid sequences of their constant domains.

Depending on the amino acid sequence of the constant region of their heavy chains, immunoglobulins can be assigned to different classes. There are five major classes of immunoglobulins: IgA, IgD, IgE, IgG and IgM, and several of these may be further divided into subclasses (isotypes), e.g. IgG-1, IgG-2, IgG-3, and IgG-4. The heavy chain constant regions hat correspond to the different classes of immunoglobulins are called α, delta, epsilon, γ, and µ, respectively. The subunit structures and three-dimensional configurations of different classes of immunoglobulins are well known.

The term "monoclonal antibody (mAb)" as used herein refers to an antibody obtained from a population of substantially homogeneous antibodies, i.e., the individual antibodies comprising the population are identical except for possible naturally occurring mutations that may be present in minor amounts. Thus, the modifier "monoclonal" indicates the character of the antibody as not being a mixture of discrete antibodies. The monoclonal antibodies include hybrid and recombinant antibodies produced by splicing a variable (including hypervariable) domain of an antibody with a constant domain (e.g. "humanized" antibodies), or a light chain with a heavy chain, or a chain from one species with a chain from another species, or fusions with heterologous proteins, regardless of species of origin or immunoglobulin class or subclass designation, as well as antibody fragments (e.g., Fab, F(ab')₂, and Fv). Cabilly, et al., U.S. Pat. No. 4,816,567; Mage & Lamoyi, in Monoclonal Antibody Production Techniques and Applications, pp.79-97 (Marcel Dekker, Inc., New York, 1987). Thus, the modifier "monoclonal" indicates the character of the antibody as being obtained from such a substantially homogeneous population of antibodies, and is not to be construed as requiring production of the antibody by any particular method.

"Humanized" forms of non-human (e.g. murine) antibodies are immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues which are found neither in the recipient antibody nor in the imported CDR or framework sequences. These modifications are made to further refine and optimize antibody performance. For "chimeric" and "humanized" antibodies see, for example, U.S. Patent No. 4,816,567; WO 91/09968; EP 452,508; and WO 91/16927).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site (antigen combining site) of an antibody (i.e. is "heterologous"), and an immunoglobulin constant domain sequence. The adhesin part of an immunoadhesin molecule typically is a contiguous amino acid sequence comprising at least the binding domain of a receptor (including cell adhesion molecules) or a ligand. The immunoglobulin constant domain sequence in the immunoadhesins may be obtained from any immunoglobulin, such as IgG-1, IgG-2, IgG-3, or IgG-4 subtypes, IgA, IgE, IgD or IgM.

Ordinarily, the C-terminus of the heterologous binding domain is fused to the N-terminus of an immunoglobulin constant region sequence, in place of the variable region(s), however N-terminal fusions are also possible.

Typically, such fusions retain at least functionally active hinge, CH2 and CH3 domains of the constant region of an immunoglobulin heavy chain. Fusions are also made to the C-terminus of the Fc portion of a constant domain, or immediately N-terminal to the CH1 of the heavy chain or the corresponding region of the light chain. This ordinarily is accomplished by constructing the appropriate DNA sequence and expressing it in recombinant cell culture. Alternatively, immunoadhesins may be synthesized according to known methods.

The precise site at which the fusion is made is not critical; particular sites are well known and may be selected in order to optimize the biological activity, secretion or binding characteristics of the immunoadhesins.

In a preferred embodiment, the C-terminus of an amino acid sequence which comprises the binding site(s) for a receptor or ligand is fused, at the N-terminal end, to the C-terminal portion of an antibody (in particular the Fc domain), containing the effector functions of an immunoglobulin, e.g. immunoglobulin G₁ (IgG-1). It is possible to fuse the entire heavy chain constant region to the sequence containing the binding site(s). However, more preferably, a sequence beginning in the hinge region just upstream of the papain cleavage site (which defines IgG Fc chemically; residue 216, taking the first residue of heavy chain constant region to be 114 [Kobet *et al.,* supra], or analogous sites of other immunoglobulins) is used in the fusion. In a particularly preferred embodiment, the amino acid sequence containing the ligand or receptor binding site(s) is fused to the hinge region and CH2, CH3; or CH1, hinge, CH2 and CH3 domains of an IgG-1, IgG-2, IgG-3, or IgG-4 heavy chain.

As used herein the phrase "bispecific immunoadhesin" designates immunoadhesins (as hereinabove defined) having at least two binding specificities, one of which may be (but does not need to be) an antigen binding site of an antibody. Bispecific immunoadhesins can generally be assembled as hetero-multimers, and particularly as hetero-dimers, -trimers or -tetramers, essentially as disclosed in WO 89/02922 (published 6 April 1989), in EP 314,317 (published 3 May 1989), and in U.S. Patent No. 5,116,964 issued 2 May 1992.

The expression "trimeric bispecific immunoadhesin" is used to designate a structure where in one arm of a Y-shaped immunoglobulin, the first heavy chain constant domain (CH1) is removed or altered to eliminate its ability to covalently bind to an immunoglobulin light chain (i.e. the light chain binding site is eliminated), while in the other arm the light chain binding site within the CH1 domain is retained, and is covalently linked to an immunoglobulin light chain.

In each arm, the heavy chain constant domain sequences are fused to "binding domains" (replacing the heavy chain variable domains) which are different from one another. One of the binding domains is heterologous (i.e. is not an antibody antigen combining site) whereas the other binding domain may be a different heterologous binding domain or a sequence comprising an antigen combining site of an antibody. The resultant structure is a trimer with two different binding specificities (heterotrimer) composed of an immunoglobulin heavy chain constant domain sequence fused to a first binding domain and a second immunoglobulin heavy chain constant domain sequence fused to a different binding domain and covalently linked to an immunoglobulin light chain. The heterologous binding domain preferably is a peptide or a polypeptide which is capable of specific binding to a native ligand or receptor (ligand or receptor binding domain). As the ultimate goal of making the heterotrimeric structure herein is to facilitate the isolation and purification of the bispecific immunoadhesin from a mixture of various immunoglobulin chain combinations, a light chain binding site is preferably retained in the heavy chain carrying a binding domain for which there is no readily available separation method (e.g. selective binding agent) available. As a result, the heavy chain in this arm of the bispecific immunoadhesin molecule will covalently bind an immunoglobulin light chain, which can be specifically recognized and separated, for example by commercially available, light chain-specific antibodies. Most preferably, a substantially intact CH1 domain is retained in this arm of the bispecific immunoadhesin molecule.

Two or more of the heterotrimers may be covalently linked to each other to provide a multimeric structure. Generally, these assembled bispecific immunoadhesins will have known unit structures. A three-chain unit may be repeated similarly to the higher molecular weight immunoglobulins, such as IgM which generally exists as a pentamer of basic four-chain units held together by disulfide bonds, or as IgA globulin, and occasionally IgG globulin, which may also exist in multimeric form in serum. In the case of multimer, each three-chain unit may be the same or different.

The term "immunoglobulin heavy chain constant domain sequence lacking a(n immunoglobulin) light chain binding site" is used to designate an immunoglobulin heavy chain constant domain sequence from which sequence elements to which the light chain is ordinarily linked are removed or sufficiently altered (mutated) so that such binding is no longer possible. In a preferred embodiment, the entire CH1 domain is deleted but shorter truncations of immunoglobulin constant domains are also suitable, provided that the section to which the light chain is ordinarily disulfide-bonded or interacts with non-covalently is removed. Alternatively, the light chain binding region of an immunoglobulin heavy chain constant domain may be mutated (by substitution or insertion) so that it is no longer capable of covalent or non-covalent binding to an immunoglobulin light chain.

The term "ligand binding domain" as used herein refers to any native cell-surface receptor or any region or derivative thereof retaining at least a qualitative ligand binding ability, and preferably the biological activity of a corresponding native receptor. In a specific embodiment, the receptor is from a cell-surface polypeptide having an extracellular domain which is homologous to a member of the immunoglobulin supergenefamily. Other typical receptors, which may or may not be members of the immunoglobulin supergenefamily but are nonetheless specifically covered by this definition, are receptors for cytokines, and in particular receptors with tyrosine kinase activity (receptor tyrosine kinases), members of the hematopoietin and nerve growth factor receptor superfamilies, and cell adhesion molecules, e. g. (E-, L- and P-) selectins.

The term "receptor binding domain" is used to designate any native ligand for a receptor, including cell adhesion molecules, or any region or derivative of such native ligand retaining at least a qualitative receptor binding ability, and preferably the biological activity of a corresponding native ligand. This definition, among others, specifically includes binding sequences from ligands for the above-mentioned receptors.

A multigene family is a group of homologous genes with similar functions. The names "supergene family" "gene superfamily" and "superfamily" refer to a set of multigene families and single-copy genes that are related by sequence homology but not necessarily in function.

Polypeptides encoded by members of the immunoglobulin supergene family (also referred to as immunoglobulin gene superfamily or immunoglobulin superfamily) contain domains with homology to constant-region domains of immunoglobulins, and include class I and class II major histocompatibility antigens, immunoglobulins and T-cell receptor α, β, γ and delta chains, such as, for example, CD1, CD2, CD4, CD8, CD28, the γ, delta and kappa chains of CD3, OX-2, Thy-1, the intracellular or neural cell adhesion molecules (I-CAM or N-CAM), lymphocyte function associated antigen-3 (LFA-3), neurocytoplasmic protein (NPC-3), poly-Ig receptor, myelin-associated glycoprotein (MAG), high affinity IgE receptor, the major glycoprotein of peripheral myelin (Po), platelet derived growth factor receptors (PDGF-A and PDGF-B), colony stimulating factor 1 (CSF-1) receptor, macrophage Fc receptor, Fc gamma receptors and carcinoembryonic antigen [Hood, L.M. *et al.*, Cell 40, 225-229 (1985)]. Homologous as defined herein means having the sequence of a member of the immunoglobulin gene superfamily or having a sequence therewith which has substantially the same as (or a greater degree of) amino acid sequence homology to a known member of the superfamily as the specific examples given above to an immunoglobulin variable or constant domain.

The term "CD4" is used to designate a native-sequence CD4 molecule [Maddon *et al.,* Cell 42, 93 (1985); WO 88/01304 published 25 February 1988], or any fragment or derivative thereof, whether isolated from natural souce, chemically synthesized or produced by methods of recombinant DNA technology, provided that they retain at least a qualitative HIV-binding ability.

The terms "lymphocyte homing receptor" and "L-selectin" are used interchangeably, and designate a native-sequence homing receptor molecule as disclosed in U.S. patent 5,098,833 issued 24 March 1992, or any fragment or derivative thereof, whether isolated from natural source, chemically synthesized or produced by methods of recombinant DNA technology, provided that they retain at least the qualitative ability of binding an L-selectin ligand, and preferably the biological activity of L-selectin.

The term "derivative" is used to define amino acid sequence and glycosylation variants, and covalent modifications of a native sequence protein, including receptor, ligand or immunoglobulin sequences that can be used in the construction of the bispecific immunoadhesins of the present invention.

The terms "amino acid" and "amino acids" refer to all naturally occurring L-α-amino acids. The amino acids are identified by either the single-letter or three-letter designations:

| | | | | | |
|---|---|---|---|---|---|
| Asp | D | aspartic acid | Ile | I | isoleucine |
| Thr | T | threonine | Leu | L | leucine |
| Ser | S | serine | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |
| Val | V | valine | Gln | Q | glutamine |
| Met | M | methionine | Asn | N | asparagine |

These amino acids may be classified according to the chemical composition and properties of their side chains. They are broadly classified into two groups, charged and uncharged. Each of these groups is divided into subgroups to classify the amino acids more accurately:
I. Charged Amino Acids
   - Acidic Residues:: aspartic acid, glutamic acid
   - Basic Residues:: lysine, arginine, histidine
II. Uncharged Amino Acids
   - Hydrophilic Residues:: serine, threonine, asparagine, glutamine
   - Aliphatic Residues:: glycine, alanine, valine, leucine, isoleucine
   - Non-polar Residues:: cysteine, methionine, proline
   - Aromatic Residues:: phenylalanine, tyrosine, tryptophan

The term "amino acid sequence variant" refers to molecules with some differences in their amino acid sequences as compared to a native amino acid sequence. The DNA sequences encoding such amino acid sequence variants are preferably capable, under stringent conditions, of hybridizing to the complement of a DNA sequence encoding the ligand binding domain of a native receptor sequence, the receptor binding domain of a native ligand sequence, a corresponding native immunoglobulin chain or the antigen combining site of a native antibody. Ordinarily, the amino acid sequence variants will possess at least about 70% homology with a receptor binding domain of a native ligand sequence, a ligand binding domain with a native receptor sequence, a native immunoglobulin sequence, or (when one of the binding domains present in the bispecific immunoadhesin is an antibody antigen combining site) with the antigen combining site of a native antibody. The homology preferably is at least about 80%, more preferably at least about 90%. The amino acid sequence variants possess substitutions, deletions, and/or insertions at certain positions within a corresponding native amino acid sequence.

"Homology" is defined as the percentage of residues in the candidate amino acid sequence that are identical with the residues in the amino acid sequence of their native counterparts after aligning the sequences and introducing gaps, if necessary, to achieve the maximum percent homology. Methods and computer programs for the alignment are well known in the art.

The "stringent conditions" are overnight incubation at 42 oC in a solution comprising: 20% formamide, 5xSSC (150 mM NaCl, 15 mM trisodium citrate), 50 mM sodium phosphate (pH 7.6), 5x Denhardt's solution, 10% dextran sulfate, and 20 µg/ml denatured, sheared salmon sperm DNA.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Insertional variants are those with one or more amino acids inserted immediately adjacent to an amino acid at a particular position in a native sequence. Immediately adjacent to an amino acid means connected to either the α-carboxy or α-amino functional group of the amino acid.

Deletional variants are those with one or more amino acids in the native amino acid sequence removed. Ordinarily, deletional variants will have one or two amino acids deleted in a particular region of the molecule.

The term "glycosylation variant" is used to refer to a glycoprotein having a glycosylation profile different from that of a native counterpart. Glycosylation of polypeptides is typically either N-linked or O-linked. N-linked refers to the attachment of the carbohydrate moiety to the side-chain of an asparagine residue. The tripeptide sequences, asparagine-X-serine and asparagine-X-threonine, wherein X is any amino acid except proline, are recognition sequences for enzymatic attachment of the carbohydrate moiety to the asparagine side chain. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be involved in O-linked glycosylation. Any difference in the location and/or nature of the carbohydrate moieties present in a ligand as compared to its native counterpart is within the scope herein.

The glycosylation pattern of native glycoproteins can be determined by well known techniques of analytical chemistry, including HPAE chromatography [Hardy, M.R. *et al.,* Anal. Biochem. 170, 54-62 (1988)] , methylation analysis to determine glycosyl-linkage composition [Lindberg, B., Meth. Enzymol*.* 28. 178-195 (1972); Waeghe, T.J. *et al.*, Carbohydr. Res. 123, 281-304 (1983)], NMR spectroscopy, mass spectrometry, etc.

The terms "binding domain variant" and "variant binding domain", that are used interchangeably, include both amino acid sequence variants and glycosylation variants of a native binding domain sequence.

"Covalent derivatives" include modifications of a native amino acid sequence with an organic proteinaceous or nonproteinaceous derivatizing agent, and post-translational modifications. Covalent modifications are traditionally introduced by reacting targeted amino acid residues of the polypeptide to be modified with an organic derivatizing agent that is capable of reacting with selected side-chains or terminal residues, or by harnessing mechanisms of post-translational modifications that function in selected recombinant host cells. The resultant covalent derivatives are useful in programs directed at identifying residues important for biological activity, for immunoassays, or for the preparation of antibodies for immunoaffinity purification of the recombinant glycoprotein. Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly alkaline conditions. Either form of these residues may be present in the polypeptides used in accordance with the present invention. Other post-translational modifications include hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)].

Covalent derivatives specifically include fusion molecules in which native or variant amino acid sequences are covalently bonded to a nonproteinaceous polymer. The nonproteinaceous polymer ordinarily is a hydrophilic synthetic polymer, i.e. a polymer not otherwise found in nature. However, polymers which exist in nature and are produced by recombinant or *in vitro* methods are useful, as are polymers which are isolated from nature. Hydrophilic polyvinyl polymers fall within the scope of this invention, e.g. polyvinylalcohol and polyvinylpyrrolidone. Particularly useful are polyvinylalkylene ethers such a polyethylene glycol, polypropylene glycol.

The binding domains may be linked to various nonproteinaceous polymers, such as polyethylene glycol, polypropylene glycol or polyoxyalkylenes, in the manner set forth in U.S. Patent Nos. 4,640,835; 4,496,689; 4,301,144; 4,670,417; 4,791,192 or 4,179,337.

The bispecific immunoadhesins of the present invention are purified to substantial homogeneity. The phrases "substantially homogeneous" "substantially homogeneous form" and "substantial homogeneity" are used to indicate that the product is substantially devoid of by-products originated from undesired immunoglobulin chain combinations. Expressed in terms of purity, substantial homogeneity means that the amount of immunoglobulin sequence containing by-products does not exceed 5 %, and preferably is below 1 %, more preferably below 0.5 %, most preferably below 0.1%, wherein the percentages are by weight.

In 1983, a retrovirus was identified in association with a syndrome characterized by a profound deficiency of cellular immune function and the occurrence of opportunistic infection and malignancy [Barre-Sinoussi, F. *et al.,* Science 220, 868-871 (1983)]. Subsequently, there have been numerous isolations of related retroviruses from patients in different geographical areas, and the etiologic role of this retrovirus has been clearly established. Whereas initially these isolates were variously referred to as lymphadenopathy associated virus (LAV), human T cell lymphotropic virus-III (HTLV-III) or AIDS associated retrovirus (ARV), today the accepted terminology is human immunodeficiency virus (HIV), which has more subtypes, e.g. HIV type-1 (HIV-1) and HIV type-2 (HIV-2), and several strains within the subtypes, such as the IIIB strain of HIV-1. The term "HIV" is used herein to include all of the foregoing retroviruses, and specifically all types and strains of the human immunodeficiency virus.

HIV is a retrovirus, containing three regions encoding structural proteins. The gag region encodes the core proteins of the virion, the pol region encodes the virion reverse transcriptase, and the env (envelope) region encodes the major glycoprotein found in the membrane of the cells infected with the HIV virus. The structural element which is believed to play a fundamental role in the pathogenesis of the virus is the env region encoding a precursor env polypeptide gp 160. Cleavage of this precursor yields gp 120 (a heavily glycosylated exterior membrane protein of about 481 amino acids) and gp 41 (a transmembrane protein of about 345 amino acids). For further details about the structure and complete nucleotide sequence of HIV-1 see, e.g. Ratner *et al.*, Nature 313, 277-285 (1985).

The specific site within the major envelope glycoprotein of HIV which is targeted for binding in accordance with the present invention preferably is within the gp 160, gp 120, gp 41 regions or on the gp 120/gp 41 complex.

In the context of the present invention the expressions "cell", "cell line", and "cell culture" are used interchangeably, and all such designations include progeny. It is also understood that all progeny may not be precisely identical in DNA content, due to deliberate or inadvertent mutations. Mutant progeny that have the same function or biological property, as screened for in the originally transformed cell, are included.

"Transformation" means introducing DNA into an organism so that the DNA is replicable, either as an extrachromosomal element or by chromosomal integration.

"Transfection" refers to the taking up of an expression vector by a host cell whether or not any coding sequences are in fact expressed.

The terms "transformed host cell" and "transformed" refer to the introduction of DNA into a cell. The cell is termed a "host cell", and it may be a prokaryotic or a eukaryotic cell. Typical prokaryotic host cells include various strains of E. coli. Typical eukaryotic host cells are mammalian, such as Chinese hamster ovary cells or human embryonic kidney 293 cells. The introduced DNA is usually in the form of a vector containing an inserted piece of DNA. The introduced DNA sequence may be from the same species as the host cell or a different species from the host cell, or it may be a hybrid DNA sequence, containing some foreign and some homologous DNA.

The terms "replicable expression vector" and "expression vector" refer to a piece of DNA, usually double-stranded, which may have inserted into it a piece of foreign DNA. Foreign DNA is defined as heterologous DNA, which is DNA not naturally found in the host cell. The vector is used to transport the foreign or heterologous DNA into a suitable host cell. Once in the host cell, the vector can replicate independently of the host chromosomal DNA, and several copies of the vector and its inserted (foreign) DNA may be generated. In addition, the vector contains the necessary elements that permit translating the foreign DNA into a polypeptide. Many molecules of the polypeptide encoded by the foreign DNA can thus be rapidly synthesized.

"Oligonucleotides" are short-length, single- or double-stranded polydeoxynucleotides that are chemically synthesized by known methods [such as phosphotriester, phosphite, or phosphoramidite chemistry, using solid phase techniques such as those described in EP 266,032, published 4 May 1988, or via deoxynucleoside H-phosphanate intermediates as described by Froehler *et al*., Nucl. Acids Res. 14, 5399 (1986)]. They are then purified on polyacrylamide gels.

### B. Starting materials

Immunoglobulins and certain variants thereof are known and many have been prepared in recombinant cell culture. For example, see U.S. Patent 4,745,055; EP 256,654; Faulkner *et al.*, Nature 298:286 (1982); EP 120,694; EP 125,023; Morrison, J. Immun. 123:793 (1979); Köhler *et al*., Proc. Nat'l. Acad. Sci. USA 77:2197 (1980); Raso *et al*., Cancer Res. 41:2073 (1981); Morrison *et al.,* Ann. Rev. Immunol. 2:239 (1984); Morrison, Science 229:1202 (1985); Morrison *et al.,* Proc. Nat'l. Acad. Sci. USA 81:6851 (1984); EP 255,694; EP 266,663; and WO 88/03559. Reassorted immunoglobulin chains also are known (see for example U.S. patent 4,444,878; WO 88/03565; and EP 68,763 and references cited therein), as are synthetic antibody binding sites (Fv analogues) produced by protein engineering [see e.g. Huston, J. S. *et al.,* Proc. Natl. Acad. Sci. USA 85, 5879-5883 (1988), and US 5,091,513 issued 25 February 1992].

The selection of the immunoglobulin that provides the heavy chain constant domain sequences either in a receptor(ligand-) immunoglobulin chimera or in an antibody, largely depends on the motivation for constructing a particular bispecific immunoadhesin herein. It is known that specific receptors on placental cells recognize the constant region of IgG (and only IgG) molecules, causing them to be ingested by endocytosis, shuttled across the cell, and released into the fetal blood on the other side of the cell. As IgG is the only immunoglobulin that can cross the placenta to travel from mother to fetus during pregnancy, IgG constant domains must be used in immunoadhesins designed for the prevention of the transmission of infections from mother to fetus. If the extension of plasma half-life of a receptor or ligand is a consideration, immunoglobulins of IgG-1, IgG-2 and IgG-4 isotypes are good candidates, as they all have *in vivo* half-lives of 21 days, as opposed to IgG-3 which has an *in vivo* half-life of 7 days. Further differences that might be advantages or detriments in certain situations are, for example, in complement activation. IgG-1, IgG-2 and IgG-3 all activate complement, however, IgG-2 is significantly weaker at complement activation than IgG-1 and does not bind to Fc receptors on mononuclear cells or neutrophils, while IgG-3 shows better complement activation than IgG-1. IgG-1 has only four serologically-defined allotypic sites, two of which (G1m1 and 2) are in the Fc portion; for two of these sites (G1m1 and 17) one allotype is non-immunogenic. In contrast, there are 12 serologically defined allotypes in IgG-3, all of which are in the Fc portion, only three of which (G3m5, 11 and 21) have an allotype which is non-immunogenic. Thus, for repeated and long-term therapeutic applications, immunoadhesins with IgG-1 derived constant domain sequences are preferred. It is possible to use immunoglobulins from different classes or isotypes in the two arms of the bispecific immunoadhesin molecule. It is further possible to combine sequences from various immunoglobulin classes or isotypes in the same arm of the molecule. For example, constructs in which the hinge of IgG-1 is replaced with that of IgG-3 are fully functional.

To prepare the bispecific immunoadhesins of the present invention, the DNA encoding a native protein from which the binding domain may be originated, may be obtained from any cDNA library prepared from tissue believed to possess mRNA for the desired protein and to express it at a detectable level. Libraries are screened with probes designed to identify the gene of interest or the protein encoded by it. For cDNA expression libraries, suitable probes usually include mono- and polyclonal antibodies that recognize and specifically bind to the desired protein; oligonucleotides of about 20-80 bases in length that encode known or suspected portions of the ligand cDNA from the same or different species; and/or complementary or homologous cDNAs or their fragments that encode the same or similar gene.

An alternative means to isolate the gene encoding a desired native protein is to use polymerase chain reaction (PCR) methodology as described in U.S. Patent No. 4,683,195, issued 28 July 1987, in section 14 of Sambrook *et al.,* Molecular Cloning: A Laboratory Manual, second edition, Cold Spring Harbor Laboratory Press. New York, 1989, or in Chapter 15 of Current Protocols in Molecular Biology, Ausubel *et al.* eds., Greene Publishing Associates and Wiley-Interscience 1991.

Another alternative is to chemically synthesize the gene encoding a desired (native or variant) protein comprising a binding domain using one of the methods described in Engels *et al.,* Agnew. Chem. Int. Ed. Engl. 28, 716 (1989). These methods include triester, phosphite, phosphoramidite and H-phosphonate methods, PCR and other autoprimer methods, and oligonucleotide syntheses on solid supports. These methods may be used if the entire nucleic acid sequence of the gene is known, or the sequence of the nucleic acid complementary to the coding strand is available, or, alternatively, if the target amino acid sequence is known, one may infer potential nucleic acid sequences, using known and preferred coding residues for each amino acid residue.

The amino acid sequence variants of the bispecific immunoadhesins of this invention are preferably constructed by mutating a DNA sequence that encodes a native protein sequence used in their construction or by starting from an earlier made variant sequence. General methods suitable for making amino acid sequence variants of polypeptides will be detailed hereinafter.

For ease, changes in the glycosylation pattern of a native glycoprotein are usually made at the DNA level, essentially using the techniques discussed hereinafter with respect to the amino acid sequence variants.

Chemical or enzymatic coupling of glycosides to amino acid sequences used in the construction of the bispecific immunoadhesins of the present invention may also be used to modify or increase the number or profile of carbohydrate substituents. These procedures are advantageous in that they do not require production of the polypeptide that is capable of O-linked (or N-linked) glycosylation. Depending on the coupling mode used, the sugar(s) may be attached to (a) arginine and histidine, (b) free carboxyl groups, (c) free sulfhydral groups such as those of cysteine, (d) free hydroxyl groups such as those of serine, threonine, or hydroxyproline, (e) aromatic residues such as those of phenylalanine, tyrosine, or tryptophan or (f) the amide group of glutamine. These methods are described in WO 87/05330 (published 11 September 1987), and in Aplin and Wriston, CRC Crit. Rev. Biochem., pp. 259-306

Carbohydrate moieties present on a native glycoprotein sequence may also be removed chemically or enzymatically. Chemical deglycosylation requires exposure to trifluoromethanesulfonic acid or an equivalent compound. This treatment results in the cleavage of most or all sugars, except the linking sugar, while leaving the polypeptide intact. Chemical deglycosylation is described by Hakimuddin *et al.,* Arch. Biochem. Biophys. 259, 52 (1987) and by Edge *et al.,* Anal. Biochem. 118, 131 (1981). Carbohydrate moieties can be removed by a variety of endo- and exoglycosidases as described by Thotakura *et al.,* Meth. Enzymol. 138, 350 (1987). Glycosylation is suppressed by tunicamycin as described by Duskin *et al.,* J. Biol. Chem. 257, 3105 (1982). Tunicamycin blocks the formation of protein-N-glycoside linkages.

Glycosylation variants of the amino acid sequences used in the construction of the bispecific immunoadhesins herein can also be produced by selecting appropriate host cells. Yeast, for example, introduce glycosylation which varies significantly from that of mammalian systems. Similarly, mammalian cells having a different species (e.g. hamster, murine, insect, porcine, bovine or ovine) or tissue (e.g. lung, liver, lymphoid, mesenchymal or epidermal) origin than the source of a native amino acid sequence, are routinely screened for the ability to introduce variant glycosylation.

### C. General Techniques

The following is a brief discussion of certain commonly used techniques of recombinant DNA technology that can be used for making the bispecific immunoadhesins of the present invention.

Further details of these and similar techniques are found in general textbooks, such as, for example, Sambrook *et al.,* Molecular Cloning: A laboratory Manual (Second Edition, Cold Spring Harbor Laboratory Press, New York, 1989; and Current Protocols in Molecular Biology, Ausubel *et al.* eds., Green Publishing Associates and Wiley-Interscience, 1991.

### Site Directed Mutagenesis

Preparation of variants of the bispecific immunoadhesins of the present invention, including sequences used in their construction, is preferably achieved by site-specific mutagenesis of DNA that encodes an earlier prepared variant or a nonvariant version of the target variant protein. Site-specific mutagenesis allows the production of variants through the use of specific oligonucleotide sequences that encode the DNA sequence of the desired mutation, as well as a sufficient number of adjacent nucleotides to provide a primer sequence of sufficient size and sequence complexity to form a stable duplex on both sides of the junction being traversed. Typically, a primer of about 20 to 25 nucleotides in length is preferred, with about 5 to 10 residues on both sides of the junction of the sequence being altered. In general, the technique of site-specific mutagenesis is well known in the art as exemplified by publications such as Adelman et al., DNA, 2: 183 (1983).

As will be appreciated, the site-specific mutagenesis technique typically employs a phage vector that exists in both a single-stranded and double-stranded form. Typical vectors useful in site-directed mutagenesis include vectors such as the M13 phage, for example, as disclosed by Messing et al., Third Cleveland Symposium on Macromolecules and Recombinant DNA, Editor A. Walton, Elsevier, Amsterdam (1981). These phage are readily commercially available and their use is generally well known to those skilled in the art. Alternatively, plasmid vectors that contain a single-stranded phage origin of replication (Veira et al., Meth. Enzymol., 153: 3 (1987)) may be employed to obtain single-stranded DNA.

In general, site-directed mutagenesis may, for example, be performed by first obtaining a single-stranded vector that includes within its sequence a DNA sequence that encodes the relevant non-mutated amino acid sequence. An oligonucleotide primer bearing the desired mutated sequence is prepared, generally synthetically, for example, by the method of Crea et al., Proc. Natl. Acad. Sci. (USA), 75: 5765 (1978). This primer is then annealed with the single-stranded non-mutated sequence-containing vector, and subjected to DNA-polymerizing enzymes such as E. coli polymerase I Klenow fragment, to complete the synthesis of the mutation-bearing strand. Thus, a heteroduplex is formed wherein one strand encodes the original non-mutated sequence and the second strand bears the desired mutation. This heteroduplex vector is then used to transform appropriate cells such as JM101 cells and clones are selected, via hybridization to a radioactive probe consisting of the ³²P-labeled mutagenesis primer, that include recombinant vectors bearing the mutated sequence arrangement.

After such a clone is selected, the mutated region may be removed and placed in an appropriate vector for the production of the desired variant, generally an expression vector of the type that typically is employed for transformation of an appropriate eukaryotic host. In the context of the present invention, Chinese hamster ovary (CHO) cells or 293 [human kidney cells described by Graham et al., J. Gen. Virol., 36: 59 (1977)] are preferred for the preparation of long-term stable polypeptide producers. However, the invention is not limited to CHO production, as it is known that numerous other cell types are suitably employed, particularly where one desires only transient production of the enzyme for test purposes.

Another method for making mutations in the DNA sequence encoding a bispecific immunoadhesin or any portion thereof involves cleaving the DNA at the appropriate position by digestion with restriction enzymes, recovering the properly cleaved DNA, synthesizing an oligonucleotide encoding the desired amino acid and flanking regions such as polylinkers with blunt ends (or, instead of using polylinkers, digesting the synthetic oligonucleotide with the restriction enzymes also used to cleave the ligand-encoding DNA, thereby creating cohesive termini), and ligating the synthetic DNA into the remainder of the structural gene encoding the bispecific immunoadhesin or any desired portion thereof.

### PCR Mutagenesis

PCR mutagenesis is also suitable for making polypeptides for practicing the present invention. While the following discussion refers to DNA, it is understood that the technique also find application with RNA. The PCR technique generally refers to the following procedure. When small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA, one of the primers is designed to overlap the position of the mutation and to contain the mutation; the sequence of the other primer must be identical to a stretch of sequence of the opposite strand of the plasmid, but this sequence can be located anywhere along the plasmid DNA. It is preferred, however, that the sequence of the second primer is located within 200 nucleotides from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions, as template copying is somewhat error-prone.

If the ratio of template to product material is extremely low, the vast majority of product DNA fragments incorporate the desired mutation(s). This product material is used to replace the corresponding region in the plasmid that served as PCR template using standard DNA technology. Mutations at separate positions can be introduced simultaneously by either using a mutant second primer or performing a second PCR with different mutant primers and ligating the two resulting PCR fragments simultaneously to the vector fragment in a three (or more)-part ligation.

### Host Cell Cultures and Vectors

Although expression in Chinese hamster ovary (CHO) cells and in the human embryonic kidney cell line 293 [Urlaub and Chasin, Proc. Natl. Acad. Sci. USA 77, 4216 (1980); Graham *et al.*, J. Gen. Virol., 36, 59 (1977)] are ultimately preferred, the vectors and methods disclosed herein are suitable for use in host cells over a wide range of eukaryotic organisms.

In general, prokaryotes are preferred for the initial cloning of DNA sequences and constructing the vectors useful in the invention. For example, E. coli K12 strain 294 (ATCC No. 31,446) and E. coli strain W3110 (ATCC No. 27,325) are particularly useful. Other suitable microbial strains include E. coli strains such as E. coli B, and E. coli X1776 (ATCC No. 31,537). These examples are, of course, intended to be illustrative rather than limiting.

Prokaryotes also are useful for expression. The aforementioned strains, as well as bacilli such as Bacillus subtilis, and other enterobacteriaceae such as, e.g., Salmonella typhimurium or Serratia marcesans, and various Pseudomonas species are examples of useful hosts for expression.

In general, plasmid vectors containing replicon and control sequences that are derived from species compatible with the host cell are used in connection with these hosts. The vector ordinarily carries a replication site, as well as marking sequences that are capable of providing phenotypic selection in transformed cells. For example, E. coli is typically transformed using pBR322, a plasmid derived from an E. coli species (see, e.g., Bolivar et al., Gene, 2: 95 (1977)). pBR322 contains genes for ampicillin and tetracycline resistance and thus provides easy means for identifying transformed cells. The pBR322 plasmid, or other microbial plasmid or phage, must also contain, or be modified to contain, promoters that can be used by the microbial organism for expression of its own proteins.

Those promoters most commonly used in recombinant DNA construction include β-lactamase (penicillinase) and lactose promoter systems (Chang et al., Nature, 375: 615 (1978); Itakura et al., Science, 198: 1056 (1977); Goeddel et al., Nature, 281: 544 (1979)) and a tryptophan (trp) promoter system (Goeddel et al., Nucl. Acids Res., 8: 4057 (1980); EPO Appl. Publ. No. 36,776), and the alkaline phosphatase systems. While these are the most commonly used, other microbial promoters have been discovered and utilized, and details concerning their nucleotide sequences have been published, enabling a skilled worker to ligate them functionally with plasmid vectors (see, e.g., Siebenlist et al., Cell, 20: 269 (1980)).

In addition to prokaryotes, eukaryotic microbes, such as yeasts, also are suitably used herein. Saccharomyces cerevisiae, or common baker's yeast, is the most commonly used among eukaryotic microorganisms, although a number of other strains are commonly available. For example, for expression in Saccharomyces, the plasmid YRp7 (Stinchcomb et al., Nature, 282: 39 (1979); Kingsman et al., Gene, 7: 141 (1979); Tschemper et al., Gene, 10: 157 (1980)) is commonly used. This plasmid already contains the trp1 gene that provides a selection marker for a mutant strain of yeast lacking the ability to grow in tryptophan, for example, ATCC No. 44,076 or PEP4-1 (Jones, Genetics, 85: 12 (1977)). The presence of the trp1 lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzeman et al., J. Biol. Chem., 255: 2073 (1980)) or other glycolytic enzymes (Hess et al., J. Adv. Enzyme Reg., 7: 149 (1968); Holland et al., Biochemistry, 17: 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In the construction of suitable expression plasmids, the termination sequences associated with these genes are also ligated into the expression vector 3' of the sequence desired to be expressed to provide polyadenylation of the mRNA and termination. Other promoters that have the additional advantage of transcription controlled by growth conditions are the promoter region for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate cells in culture (tissue culture) has become a routine procedure in recent years [Tissue Culture, Academic Press, Kruse and Patterson, editors (1973)]. Examples of such useful host cell lines are VERO and HeLa cells, CHO cell lines, and W138, BHK, COS-7, (ATCC CRL 1651), 293, and MDCK (ATCC CCL 34) cell lines. Expression vectors for such cells ordinarily include (if necessary) an origin of replication, a promoter located in front of the gene to be expressed, along with any necessary ribosome binding sites, RNA splice sites, polyadenylation sites, and transcriptional terminator sequences.

For use in mammalian cells, the control functions on the expression vectors are often provided by viral material. For example, commonly used promoters are derived from polyoma, Adenovirus2, and most frequently Simian Virus 40 (SV40). The early and late promoters of SV40 virus are particularly useful because both are obtained easily from the virus as a fragment that also contains the SV40 viral origin of replication (Fiers et al., Nature, 273: 113 (1978)). Smaller or larger SV40 fragments are also suitably used, provided there is included the approximately 250-bp sequence extending from the HindIII site toward the BglI site located in the viral origin of replication. Further, it is also possible, and often desirable, to utilize promoter or control sequences normally associated with the desired gene sequence, provided such control sequences are compatible with the host cell systems.

An origin of replication typically is provided either by construction of the vector to include an exogenous origin, such as may be derived from SV40 or other viral (e.g., Polyoma, Adeno, VSV, BPV) source, or by the host cell chromosomal replication mechanism. If the vector is integrated into the host cell chromosome, the latter is often sufficient.

Satisfactory amounts of the bispecific immunoadhesins are produced by cell cultures; however, refinements, using a secondary coding sequence, serve to enhance production levels even further. The secondary coding sequence comprises dihydrofolate reductase (DHFR) that is affected by an externally controlled parameter, such as methotrexate (MTX), thus permitting control of expression by control of the MTX concentration.

In the selection of a preferred host cell for transfection by the vectors of the invention that comprise DNA sequences encoding both the target polypeptide and DHFR protein, it is appropriate to consider the type of DHFR protein employed. If wild-type DHFR protein is employed, it is preferable to select a host cell that is deficient in DHFR, thus permitting the use of the DHFR coding sequence as a marker for successful transfection in selective medium that lacks hypoxanthine, glycine, and thymidine. An appropriate host cell in this case is the CHO cell line deficient in DHFR activity, prepared and propagated, as described by Urlaub and Chasin, Proc. Natl. Acad. Sci. (USA) 77: 4216 (1980).

On the other hand, if DHFR protein with low binding affinity for MTX is used as the controlling sequence, it is not necessary to use DHFR-deficient cells. Because the mutant DHFR is resistant to MTX, MTX-containing media can be used as a means of selection, provided that the host cells are themselves MTX sensitive. Most eukaryotic cells that are capable of absorbing MTX appear to be sensitive to MTX. One such useful cell line is a CHO line, CHO-K1 (ATCC No. CCL 61).

### Typical Cloning and Expression Methodologies Available

If mammalian cells are used as host cells, transfection generally is carried out by the calcium phosphate precipitation method as described by Graham and Van der Eb, Virology, 52: 546 (1978). However, other methods for introducing DNA into cells such as nuclear injection, electroporation, or protoplast fusion are also suitably used.

If yeast are used as the host, transfection is generally accomplished using polyethylene glycol, as taught by Hinnen, Proc. Natl. Acad. Sci. U.S.A., 75: 1929-1933 (1978).

If prokaryotic cells or cells that contain substantial cell wall constructions are used, the preferred method of transfection is calcium treatment using calcium as described by Cohen et al., Proc. Natl. Acad. Sci. (USA) 69: 2110 (1972), or more recently electroporation.

Construction of suitable vectors containing the desired coding and control sequences employs standard ligation techniques. Isolated plasmids or DNA fragments are cleaved, tailored, and religated in the form desired to form the plasmids required.

Cleavage is performed by treating with restriction enzyme (or enzymes) in suitable buffer. In general, about 1 µg plasmid or DNA fragments is used with about 1 unit of enzyme in about 20 µl of buffers and substrate amounts for particular restriction enzymes are specified by the manufacturer.) Incubation times of about one hour at 37°C are workable. After incubation, protein is removed by extraction with phenol and chloroform, and the nucleic acid is recovered from the aqueous fraction by precipitation with ethanol.

If blunt ends are required, the preparation may be treated for 15 minutes at 15°C with 10 units of the Klenow fragment of DNA Polymerase I (Klenow), phenol-chloroform extracted, and ethanol precipitated.

Size separation of the cleaved fragments is performed using 6 percent polyacrylamide gel described by Goeddel et al., Nucleic Acids Res., 8: 4057 (1980).

For ligation, approximately equimolar amounts of the desired components, suitably end tailored to provide correct matching, are treated with about 10 units T4 DNA ligase per 0.5 µg DNA. (When cleaved vectors are used as components, it may be useful to prevent religation of the cleaved vector by pretreatment with bacterial alkaline phosphatase.)

For analysis to confirm correct sequences in plasmids constructed, the ligation mixtures are typically used to transform E. coli K12 (ATCC 31,446) or other suitable E. coli strains, and successful transformants selected by ampicillin or tetracycline resistance where appropriate. Plasmids from the transformants are prepared and analyzed by restriction mapping and/or DNA sequencing by the method of Messing et al., Nucleic Acids Res., 9: 309 (1981) or by the method of Maxam et al., Methods of Enzymology, 65: 499 (1980).

After introduction of the DNA into the mammalian cell host and selection in medium for stable transformants, amplification of DHFR-protein-coding sequences is effected by growing host cell cultures in the presence of approximately 20,000-500,000 nM concentrations of MTX, a competitive inhibitor of DHFR activity. The effective range of concentration is highly dependent, of course, upon the nature of the DHFR gene and protein and the characteristics of the host. Clearly, generally defined upper and lower limits cannot be ascertained. Suitable concentrations of other folic acid analogs or other compounds that inhibit DHFR could also be used. MTX itself is, however, convenient, readily available, and effective.

### D. Use of the bispecific immunoadhesins

Utilizing the preparation and purification scheme disclosed in the present application, bispecific immunoadhesins can be purified to a degree suitable for therapeutic applications. The actual therapeutic use will depend on the functionalities of any particular bispecific immunoadhesin.

The therapeutic potentials of the (CD4 x anti-CD3) bispecific molecules have already been discussed, and further details will be provided hereinafter. (CD4 x anti-CD16) bispecific immunoadhesins are expected to have a similar therapeutic utility.

TNFR-IgG x anti-endotoxin antibody (TNFR x anti-endotoxin) immunoadhesins can, for example, be used for the prevention or treatment of endotoxic shock.

Bispecific immunoadhesins having the antigen binding site specific for fibrin linked to a protein comprising the active portion of a plasminogen activator, such as streptokinase, urokinase, prourokinase or t-PA, are useful as immunotherapeutics. Through their fibrin-specific binding site, such molecules are capable of localizing at the site of a thrombus, and the thrombus is lysed by the enzyme activity of the plasminogen activator portion of the hybrid molecule. (See WO 88/03559 published 19 May 1988.)

(CD4 x L-selectin) bispecific immunoadhesins combine the lymph node binding function of L-selectin and the HIV binding function of CD4. Because this bispecific molecule binds to endothelial tissue not only in lymph nodes but in secondary lymphoid organs such as Peyer's patches and in the brain, it may be used for delivery of CD4-IgG across the blood-brain barrier for the treatment of HIV-related dementia.

For therapeutic applications, the bispecific immunoadhesins are combined with a pharmaceutically acceptable carrier. Such carriers are well known in the art and are disclosed, for example, in Remington's Pharmaceutical Sciences, 16th Edition, 1980, Mac Publishing Company. The determination of the therapeutically efficient concentration for any desired bispecific immunoadhesin is well within the skill of an ordinary artisan. The selection of a preferred pharmaceutical formulation and the determination of the therapeutically efficient dose regimen are facilitated by information available for the two functionalities when used as therapeutics alone or in the form of a monospecific immunoadhesin. As a general rule, the bispecific immunoadhesins are expected to be efficacious at lower concentrations than the respective proteins from which their binding domains are derived.

The bispecific immunoadhesins of the present invention may, for example, be placed into sterile, isotonic formulations together with required cofactors. The formulation is preferably liquid or may be lyophilized powder. The (CD4 x anti-CD3) bispecific immunoadhesins can, for example, be diluted with a formulation buffer comprising 5.0 mg/ml citric acid monohydrate, 2.7 mg/m trisodium citrate, 41 mg/ml mannitol, 1 mg/ml glycine and 1 mg/ml polysorbate 20. This solution can be lyophilized, stored under refrigeration and reconstitued prior to administration with sterile Water-For-Injection (USP).

The bispecific immunoadhesins may also be administered via microspheres, liposomes, other microparticulate delivery systems or sustained release formulations placed in certain tissues including blood. Suitable examples of sustained release carriers include semipermeable polymer matrices in the form of shaped articles, e.g. suppositories, or microcapsules. Implantable or microcapsular sustained release matrices include polylactides (U.S. Patent No. 3,773,919; EP 58,481), copolymers of L-glutamic acid and gamma ethyl-L-glutamate [Sidman *et al.,* Biopolymers 22(1), 547-556 (1985)], poly(2-hydroxyethyl-methacrylate) or ethylene vinyl acetate (Langer *et al.,* J. Biomed. Mater. Res. 15, 167-277 (1981); Langer, Chem. Tech. 12, 98-105 (1982)].

Liposomes containing the bispecific immunoadhesins can be prepared by well known methods: DE 3,218,121; Epstein *et al.,* Proc. Natl. Acad. Sci. USA 82, 3688-3692 (1985); Hwang *et al.*, Proc. Natl. Acad. Sci. USA 77, 4030-4034 (1980); EP 52322; EP 36676; EP 88046; EP 143,949; EP 142,541; U.S. Patents Nos. 4,485,045 and 4,544,545. Ordinarily the liposomes are of the small (about 200-800 Angstroms) unilamellar type in which the lipid content is greater than about 30 mol. % cholesterol, the selected proportion being adjusted for the optimal rate of the polypeptide leakage.

It may be advantageous to attach the bispecific immunoadhesins of the present invention to liposomes containing toxins or various therapeutic agents. For example, (CD4 x anti-CD3) bispecific immunoadhesins can be covalently linked to liposomes comprising toxins or agents which can inhibit HIV replication [Matsukura *et al*., Proc. Natl. Acad. Sci. USA 86, 4244-4248 (1989)]. Via their CD4 arm, the bispecific immunoadhesins inhibit the interaction of HIV with the CD4 receptor and target the toxins or anti-HIV agents to HIV infected cells, whereas their anti-CD3 arm is instrumental in activating the cytotoxic T lymphocytes, thereby recruiting the cellular immune response for fighting HIV infection. The attachment of the bispecific immunoadhesins to liposomes may be performed by any known cross-linking agent, such as heterobifunctional cross-linking agents that have been widely used for their regiospecificity in coupling toxins or chemotherapeutics to antibodies for targeted delivery. The linkage of CD4 via its carbohydrate moieties rather than its free amino groups is preferred, as such linkage has been desmonstrated to enable the conjugation of CD4 to other compounds with no loss in its gp 120 binding affinity (an effect which occurs with amino group-directed reagents). Soluble CD4 (sCD4) has been successfully conjugated via its carbohydrate moieties to liposomes, using a novel carbohydrate-directed cross-linking reagent: 4-(4-maleimidophenyl)butyric acid hydrazide (MPBH) [Duzgunes, N., Flasher, D., Chamow, S., Ashkenazi, A., Dazin, P., and Konopka, K. J. Cell. Biochem. Abst. Suppl. 16E 77 (1992); Chamow *et al*., J. Biol. Chem. 267, 15916-15922 (1992). A similar strategy can be employed for attaching other bispecific immunoadhesins to liposomes.

Bispecific immunoadhesins can be used also as diagnostic tools where one arm of the bispecific molecule is a targeting arm, and the other is a reporting arm (directly or detected by a reporter molecule). For example, the CD4 arm is capable of targeting a (CD4 x anti-CD3) bispecific immunoadhesin to HIV-infected cells, whereas the anti-CD3 portion can be used for detection. Similarly, the bispecific immunoadhesins combining plasminogen activator and fibrin-binding functionalities may be used in immunodiagnostic applications, including *in vivo* immunodiagnosis, by detectably labeling the bispecific molecule with a radionuclide.

### E. Preferred embodiments

In a preferred embodiment, the DNA sequences encoding the two immunoglobulin heavy chains (whether fusions of heterologous, e.g. receptor or ligand binding domains withy immunoglobulin heavy chain constant domain sequences or native or chimeric antibody heavy chain sequences including a desired antibody antigen combining site) and the immunoglobulin light chain are inserted in separate vectors and are cotransfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment, the bispecific immunoadhesins of the present invention comprise an amino acid sequence specifically binding to a foreign antigen on the surface of a target cell, and preferably to an integral viral protein of a retrovirus, preferably HIV. The binding site preferably is within the gp 160, gp 120, gp 41 region of the major envelope glycoprotein of HIV, including the gp 120/gp 41 complex.

The HIV-binding domain preferably is a CD4 amino acid sequence. The amino acid sequence of the native CD4 molecules is known [Maddon *et al.,* Cell 42, 93 (1985); WO 88/01304 published 25 February 1988]. Several approaches have been used to define the amino acids within the V1 region of the CD4 amino acid sequence to which the gp 120 of the HIV virus binds. These approaches include the technique of random saturation mutagenesis coupled with selection of escape mutants [Peterson and Seed, Cell 54, 65 (1988) and EP 341,444 published 15 November 1989], and homolog-scanning mutagenesis (replacement of sequences from human CD4, which binds gp 120, with unconserved sequences from mouse CD4, which does no bind gp 120) [Landau *et al.,* Nature 334, 159 (1988); Clayton *et al.,* Nature 335, 363 (1988); and WO 89/0322, published 20 April 1989].

As the transmembrane and cytoplasmic regions are not required for the gp 120 binding of CD4, these domains may be deleted to provide a soluble CD4 molecule. Usually at least residues 368 to 395 (the transmembrane domain), and frequently also residues 396 to 433 (the cytoplasmic domain) are deleted.

Insertional variants of CD4 were, for example, published by Mizukami *et al.,* Proc. Natl. Acad. Sci. USA 85, 9273 (1988). Clayton *et al.,* Nature 335, 363 (1988) disclose CD4 variants with individual and multiple substitutions corresponding to regions of the nurine CD4 molecule. Synthetic peptide fragments of CD4 were published by Lifson *et al.,* Science 241, 712 (1988). Peterson and Seed, supra generated several substitution or deletion variants of native CD4 by random mutagenesis. Further CD4 amino acid sequence variants and covalent derivatives are, for example, disclosed in WO 89/02922 published 6 April 1989. Specifically, an amino acid can be inserted adjacent to, deleted from, or substituted for an amino acid of CD4 at positions corresponding to position 7, 15, 17, 21, 22, 23, 28, 29, 30, 32, 35, 36, 37, 39, 40, 44, 45, 46, 49, 51, 52, 53, 54, 57, 58, 59, 62, 63, 64, 75, 77, 78, 79, 80, 81, 82, 85, 87, 89, 91, or 94 of the native CD4 amino acid sequence. [See also Ashkenazi *et al.,* Proc. Natl. Acad. Sci. USA 87, 10535-10539 (1990).]

Immunoadhesins comprising the fusion of CD4 with immunoglobulin sequences of IgG or IgM subtype are known in the art [Byrn, R.A., 1990, supra; Capon, D.J., 1989, supra; Traunecker, A., Nature 339, 68-70 (1989)], and are disclosed in WO 89/02922 published 6 April 1989; and EP 314,317, supra.

Cell lines producing mouse anti-human CD3 antibodies, including their antigen recognition and binding sites (Fv-CD3) are known in the art and are, for example, disclosed in Berg *et al.,* supra; Traunecker *et al.,* supra and Huston *et al.*, Proc. Natl. Acad. Sci. USA 85, 5897-5899 (1988). Methods for making biosynthetic antibody antigen binding sites are, for example, disclosed in U.S. Patent No. 5,091,513 issued 25 February 1992.

The (CD4 x anti-CD3) bispecific immunoadhesins of the present invention can be made following general procedures disclosed in WO 89/02922 (disclosing CD4-Ig immunoadhesins) and WO 91/08298 published 13 June 1991 (disclosing immunoadhesins comprising adhesons that are not members of the immunoglobulin gene superfamily).

In a preferred embodiment, the CD4-anti-CD3 bispecific immunoadhesins herein are constructed by transfecting mammalian cells with plasmids encoding, individually, human CD4-IgG (preferably IgG-1 or IgG-3), humanized mouse anti-CD3 γ1 or γ3 heavy chain, and humanized mouse anti-CD3 kappa light chain. Cells transfected in this way produce and assemble these polypeptides in three oligomeric combinations: (i) a heterotetramer of two IgG heavy and two IgG light chains, which contains two binding sites for CD3; (ii) a homodimer of two CD4-IgG fusion proteins, which contains two binding sites for gd 120; and (iii) a heterotrimer of one IgG light chain, one IgG heavy chain and one CD4-IgG, which contains one binding site for CD3 and one for gp 120 (the bispecific immunoadhesin).

Various ratios of the vectors carrying the DNA encoding CD4-IgG-1, humanized mouse anti-CD3 γ1 heavy chain, and humanized mouse anti-CD3 kappa light chain, respectively, were tried in order to increase the yield of the target bispecific immunoadhesin. It was found that if these three vectors were introduced into transfected cells in a ratio of 60:20:20 percent CD4-IgG/anti-CD3 light chain/anti-CD3 heavy chain, the amount of bispecific immunoadhesin secreted by the cells was about two-fold greater than that secreted when the ratio was 33:33:33 percent, and about 20-fold greater than the amount secreted when a ratio of 10:45:45 was used (see Figure 2). Accordingly, equally good results are expected by inserting the CD4-immunoglobulin chimera encoding DNA sequence in one vector, and the anti-CD3 antibody heavy and light chain encoding DNA sequences in another vector, and altering the mutual ratios of the two vectors to produce the three chains in the desired 60:20:20 percent ratio. Although this ratio represents the best mode as currently contemplated, further improvements might be achieved by testing additional ratios. A percentage ratio between 70:20:10 and 60:20:20, preferably between 60:30:10 and 60:20:20 of CD4-IgG/anti-CD3 light chain/anti-CD3 heavy chain is expected to provide the best yields for the desired trimeric bispecific immunoadhesin. The optimal ratio can be determined using similar strategies for any other specific immunoadhesin.

The foregoing three products [products (i)-(iii)] are preferably isolated from the cell culture supernatants by affinity chromatography based on their Fc portion. The isolation is preferably performed on a Protein A Sepharose chromatographic column which binds Fc-bearing proteins. After elution, the proteins comprising a CD4 portion may be separated from the anti-CD3 heterotetramer by immunoaffinity chromatography using an immobilized anti-CD4 monoclonal antibody. From the CD4-containing eluate (containing the bispecific trimer and the CD4-IgG dimer) the bispecific (CD4 x anti-CD3) molecule can be removed by affinity chromatography based on its light chain portion, using an immobilized anti-human kappa IgG polyclonal antibody. If desired, any of the separation/isolation steps may be repeated. The isolated bispecific immunoadhesin may be further purified by known techniques, such as ion exchange chromatography.

The CD4 portion-based and immunoglobulin light chain-based separation steps can also be performed in a reverse order, first separating the two immunoglobulin light chain-bearing products from the CD4-IgG homodimer, and subsequently isolating the (CD4 X anti-CD3) bispecific immunoadhesin from the anti-CD3-Ig homotetramer (composed of two heavy chain-light chain pairs), based on its CD4 portion.

The purified (CD4 x anti-CD3) bispecific immunoadhesins are formulated into conventional pharmaceutical formulation using pharmacologically acceptable excipients [see Remington's Pharmaceutical Sciences 16th Ed. 1980, Mac Publishing Company] . The preferred formulation is liophilized powder, which may be diluted with a liquid diluent, preferably sterile water to any desired stock concentration. Dilutions suitable for intravenous administration may be made with conventional formulations buffers and/or stabilizers immediately prior to administration.

The CD4 x anti-CD3 antibody bispecific immunoadhesins are administered to patients at high risk of or having HIV infection at a dosage capable of activating a CTL response against HIV-infected cells, but not uninfected cells. These bispecific immunoadhesins are particularly suitable for the prevention of the establishment of HIV infection in an uninfected individual that has been or is at risk to be exposed to HIV, or in a recently infected individual in which the HIV infection is still limited to a relatively small number of cells. As bispecific (CD4 x anti-CD3) immunoadhesins constructed with an IgG heavy chain are expected to cross the human placenta similarly to IgG immunoglobulins and CD4-IgG immunoadhesins, they are particularly suitable for the prevention of maternal/fetal transmission of HIV infection. For this indication, the bispecific immunoadhesin is preferably administered to HIV seropositive women at the onset of labor, more preferably about one to two weeks before the labor and at the onset of labor. Alternatively or in addition, the (CD4 x anti-CD3) bispecific immunoadhesin can also be administered to the infants postnatally. The preferred route of administration is injection or intravenous infusion, and the ordinary dosage is about 1 mg/kg to about 6 mg/kg i.v. bolus. This administration is expected to reduce the load of HIV in the mother prior to birth and thus the risk of transmission. In addition, transfer of the bispecific immunoadhesin to the fetus or postnatal administration to the infant can provide CTL-mediated protection for the newborn baby.

The CD4-Ig x L-selectin-Ig (CD4 x L-selectin) bispecific immunoadhesins represent a different strategy for the prevention or treatment of HIV infection. The selectins are unified structurally by the inclusion of lectin, egf-like and complement binding-like domains, and functionally by their ability to mediate cell binding through interactions between their lectin domains and cell surface carbohydrate ligands. There are three members identified so far in the selectin family of cell adhesion molecules: L-selectin (a.k.a. peripheral lymph node homing receptor (pnHR), LEC-CAM-1, LAM-1, gp90^{MEL}, gp100^{MEL}, gp110^{MEL}, MEL antigen, Leu-8 antigen, TQ-1 antigen, DREG antigen) ; E-selectin (LEC-CAM-2, LECAM-2, ELAM-1) and P-selectin (LEC-CAM-3, LECAM-3, GPM-140, PADGEM). L-selectin (see U.S. Patent No. 5,098,833 issued 24 March 1992L is found on leukocytes and is involved with the trafficking of lymphocytes to peripheral lymphoid tissues [Gallatin *et al.,* Nature 303, 30-34 (1983)] and with acute neutrophil-mediated inflammatory responses [Watson *et al.,* Nature 349, 164-167 (1991)]. (CD4 x L-selectin) bispecific immunoadhesins combine the lymph node homing ability of L-selectin with the ability of CD4 to bind HIV. By targeting the CD4 molecules to the lymph nodes these bispecific molecules can provide high local concentrations of CD4 at a location where HIV infection is most widespread.

The L-selectin portion of the (CD4 x L-selectin) bispecific immunoadhesin molecule may be native-sequence L-selectin as disclosed in U.S. patent No. 5,098,833, supra or any fragment or derivative thereof retaining the qualitative lymph node binding function of native L-selectin. Such derivatives are disclosed in the cited U. S. patent, and preferably are encoded by a DNA sequence capable of hybridizing, under stringent conditions, with the lectin domain of L-selectin. Immunoadhesins comprising the fusion of an L-selectin sequence with an immunoglobulin constant domain sequence are disclosed in U. S. patent No. 5,116,964 issued 26 May 1992. In these an N-terminal portion of an L-selectin (referred to as LHR), which contains the binding site for the endothelium of lymphoid tissue, is fused to the C-terminal Fc portion of an immunoglobulin. In a particular embodiment, the entire heavy chain constant region of an IgG immunoglobulin is fused to a portion of the L-selectin molecule. In another embodiment, a sequence beginning in the hinge region just upstream of the papain cleavage site which defines IgG Fc chemically (residue 216, taking the first rresidue of heavy chain constant region to be 114 (Kabat *et al.*, supra), or analogous sites of other immunoglobulins, is fused to a portion of the LHR. Specific murine L-selectin (MLHR)-IgG chimeras containing the lectin, lectin-egf, and lectin-egf-complement regulatory motifs of L-selectin are disclosed in Example 4 and shown in Figure 8 of U. S. patent No. 5,116,964, supra. This patent also discloses the construction of heterodimeric CD4-IgG-MLHR-IgG chimeras, in which the CH1 domain and a portion of the hinge region up to the first cysteine residue was removed in each arm of the molecule. Expression in human 293 cells resulted in three molecules: CD4-IgG homodimers, LHR-IgG homodimers, and CD4-IgG-LHR-IgG heterodimers.

According to the present invention, the (CD4 x L-selectin) bispecific immunoadhesins is designed to have a heterotrimeric structure as hereinabove described. Such heterotrimers are composed of a CD4-immunoglobulin heavy chain constant domain fusion protein in one arm, and an L-selectin-immunoglobulin heavy chain constant domain sequence associated with an immunoglobulin light chain, in the other arm. These asymmetic bispecific molecules can be prepared and purified essentially as described for (CD4 x anti-CD3) bispecific immunoadhesins.

The present bispecific immunoadhesin therapy can also be combined with other therapies useful for the prevention or treatment of HIV infection and related conditions, and in particular for slowing down the progression of the development of HIV infection. Combination therapy may, for example, be performed by using a liposome drug delivery system. As hereinabove described, bispecific immunoadhesins can be covalently linked to liposomes containing any toxin or drug. The attachment of bispecific immunoadhesins designed for use in anti-HIV herapy to liposomes containing drugs with anti-HIV activity provides a new means of combination therapy. For CD4 it has already been demonstrated that its linkage via its carbohydrate portion to a liposome does not interfere with its ability to bind HIV (see Duzgunes, N. *et al*., supra).

Further details of the invention will be apparent from the following non-limiting examples.

### Example 1

### Plasmid construction

The CD4-IgG-1 chimera was constructed as described in Byrn *et al*., Nature 344, 667 (1990). Briefly, this construct consists of residues 1-180 of the mature human CD4 protein fused to human IgG-1 sequences beginning at aspartic acid 216 (taking amino acid 114 as the first residue of the heavy chain constant region [Kabat *et al.,* supra]) which is the first residue of the IgG-1 hinge after the cysteine residue involved in heavy-light chain bonding, and ending with residue 441. This molecule contains the CD4 V1 and V2 domains, linked to the hinge and Fc (CH2 and CH3) domains of human IgG-1, and is designated CD4₂Fc1. The construction of humanized anti-CD3 heavy chain and humanized anti-CD3 light chain is described in Shalaby *et al.,* J. Exp. Med. 175, 217 (1992). The DNA sequences encoding these three constructs were inserted individually into the mammalian expression vector pRK5 (EP 307,247 published 15 March 1989).

### Transfections

The CD4-IgG-1 (CD4₂Fc1), anti-CD3 heavy chain and anti-CD3 light chain expression vectors were introduced into human embryonic kidney 293S cells by transient transfection, using a modification of the calcium phosphate precipitation method [Gorman, C. in DNA Cloning Vol. III (ed. Glover, D.M.), pp. 143-190, IRL, Oxford, 1985]. The total amount of plasmid DNA transfected was 20 µg, and the amount of each individual plasmid was varied to assess the effect of the relative amounts of DNA on the relative amounts of the possible protein products.

The three plasmids encoding the CD4-IgG and the anti-CD3 heavy and light chain constructs were transfected in various combinations (total DNA amount of 20 µg), and the amount of protein containing the immunoglobulin Fc region in serum-free supernatants from metabolically labeled, transfected cells was assessed by radioimmunoprecipitation with protein A (Pansorbin) (Figure 2).

In the absence of all three plasmids, no Fc-containing proteins were detectable (rightmost lane). In the presence of both anti-CD3 plasmids and in the absence of the CD4-IgG plasmid, only one major band was observed which barely, migrated into the gel, representing the anti-CD3 heterotetrameric antibody (second lane from right). Similarly, addition of the CD4-IgG plasmid alone resulted in a single band, representing the CD4-IgG immunoadhesin (leftmost lane). Addition of the CD4-IgG plasmid (10%) with the two anti-CD3 plasmids (45% each), resulted in a new band corresponding to the desired bispecific immunoadhesin. The amount of this protein increased as the amount of CD4-IgG plasmid input was increased; the highest amount observed in this particular experiment was produced at a 60:20:20 % ratio of the CD4-IgG and the two anti-CD3 plasmids. These results show that the amount of the desired product can be altered by changing the amounts of the DNAs transfected. Although the 60:20:20 % ratio produced the desired (CD4 x anti-CD3) bispecific trimer in a high concentration, further improvement may be possible by altering the plasmid ratios used for transfection.

### Example 2

### Recovery and purification of the (CD4 x anti-CD3) bispecific antibody

The product mixture obtained with the 60:20:20% plasmid ratio was treated as follows.

### Affinity chromatography based on the Fc portion

Harvested cell culture supernatant (3.5 L) was loaded onto Protein A Sepharose (10 mL) which had been equilibrated in 10 mM Tris pH 7.4, 150 mM NaCl. After washing in the same buffer to remove unbound material, bound Fc-bearing proteins (17.5 mg) were eluted in 50 mM citric acid pH 3.0, 20% (w/v) glycerol. This pool was neutralized by addition of 3 M Tris pH 9.0.

### Affinity Chromatography based on CD4 portion

To first remove the anti-CD3 heterotetramer from the mixture containing the CD4-IgG x anti-CD3 heavy chain - light chain pair heterotrimer (the desire bispecific compound) and the homodimer of two CD4-IgG heavy chains, the Protein A-eluted pool was loaded onto an anti-CD4 immunoaffinity column. We had previously identified a monoclonal antibody (mAb 754) that efficiently bound and eluted CD4-IgG under mild conditions. Other anti-CD4 antibodies, such as Leu 3A (Becton Dickinson), are commercially available and are equally suitable for this purification step. MAb 754 (5 mg) was immobilized onto controlled pore glass (1 mL), equilibrated in 20 mM Tris pH 7.4, 150 mM NaCl, and Protein A pool (1 mg) was loaded. Unbound material was washed out with the same buffer, and material non-specifically bound to the matrix was washed out with 20 mM Tris pH 7.4, 150 mM NaCl, 0.5 M tetramethylammonium chloride (TMAC). Bound protein was elute with 50 mM Tris pH 7.4, 3.5 M MgCl₂. The mAb 754 column was cycled 11 types to produce a total of 4.8 mg of the trimeric (CD4 x anti-CD3) bispecific protein.

### Affinity chromatography based on kappa light chain

To recover the (CD4 x anti-CD3) heterotrimer free of the CD4-IgG homodimer, the mAb 754 pool was loaded onto a second immunoaffinity column, this one specific for human kappa chain. A polyclonal anti-human kappa IgG (3 mg, Sigma K-3502) was immobilized onto controlled pore glass (1 mL), equilibrated, loaded (0.5 mL) and washed as described above for mAb 754. In this case, bound, purified bispecific immunoadhesin was eluted with 50 mM citric acid pH 3.0, 20% (w/v) glycerol, and neutralized as before. The column was cycled 6 times to produce a total of 0.83 mg of the (CD4 x anti-CD3) heterotrimer.

### Results

By altering the ratio of the three plasmids used for transfection the reaction equilibrium was shifted in favor of the desired bispecific product, facilitating its separation and recovery. When the percentage ratio of the plasmids encoding the CD4-IgG fusion, the anti-CD3 antibody heavy chain and the anti-CD3 antibody light chain, respectively was 60:20:20, the approximate (CD4 x anti-CD3) heterotrimer/CD4-IgG homodimer/anti-CD3 heterotetramer ratio was 40:40:20% (estimate based on staining intensities), and the (CD4 x anti-CD3) bispecific immunoadhesin was isolated in a yield of about 14 to 20%. Concievably, further improvement in product ratios and yield can be achieved by testing additional plasmid ratios, and by carrying out the CD4-based and the immunoglobulin light chain-based purification steps in a reverse order.

### Example 3

### Lysis of HIV-1 infected cells by effector cell retargeting with humanized antibody, CD4-anti-CD3-IgG

### Materials and Methods:

Cell lines. Human T cell lines CEM, HIV-I IIIB chronically infected (CEM/IIIB) or uninfected (CEM), were cultured in RPMI 1640 medium (JRH Biosciences, Lenexa,KS) supplemented with 10% heat-inactivated fetal calf serum (growth medium, GM). About 95% of cells express HIV-1 gp120 by immunofluorescence assay with anti-HIV-1 serum. CEM is CD4⁺ cell line with minimal or no expression of CD3 antigen [Kurrle R. Cluster report: CD3 in Knapp W. (ed.) Leucocyte Typing IV (Oxford: Oxford University press), 290-293 (1989)], and is not detected de novo after HIV infection [Bodeus, M. *et al.,* Modification of CEM Surface Phenotype During HIV Infection in Knapp, W. (ed.) Leucocyte Typing IV (Oxford: Oxford University press) 382-383 (1989)].

Antibodies. The bifunctional immunoadhesin was prepared by coexpressing human anti-CD3 antibody with CD4-IgG immunoadhesin [Byrn, R.A. *et al*., Nature 334:667-670 (1990)] as described in Example 1. The human monoclonal antibody, F105, was provided by Dr. Marshall Posner, New England Deaconess Hospital [Posner, M.R. *et al.,* J. Immunol. 146:4325-4332 (1991)]. This IgG1 antibody reacts with HIV-1 gp120 and blocks the binding of gp120 to CD4.

Effector cells. Peripheral blood lymphocytes (PBL) were isolated from blood from normal donors by standard Ficoll-Hypaque gradient centrifugation and maintained in GM as described [Junghans, R.P. *et al.,* Cancer Immunol. Immunother, 31:207-212 (1990)]. PBL stimulated with interleukin 2 (IL-2) were cultured in the presence of 1000 U/ml affinity-purified human recombinant IL-2 (Hoffmann-LaRoche, Inc.) for 14-16 hours. Human cytotoxic lymphocytes (CTL) were a gift of Dr. John Yanelli (NCI, Bethesda), and were prepared and activated according to standard procedures [Yannelli, J.R. *et al.,* J. Immunol. Methods 139:1-16 (1991)].

Effector cell retargeting (ECR). ECR was performed as described [Junghans, R.P., Supra] with the following modifications. 2x10⁶ CEM and CEM/IIIB cells were labelled with ⁵¹Cr at 0.5 mCi/2ml GM overnight at 37°C, washed 4 times with GM, and suspended at 1x10⁵ cells/ml. The cells were incubated with the indicated concentrations of antibodies at room temperature for 1 hour. Effector cells prepared the preceding day were spun down and resuspended in GM at 1.5x10⁶ cells/ml. 50 ul of ⁵¹Cr-labelled target cells (5000 cells) were mixed with 100 ul of effector cells (150000 cells) at an effector:target ratio of 30:1. All mixtures were tested in triplicate or quadruplicate in 96-well U-bottom plates. The plates were incubated at 37°C for 3 hours. Supernatants were harvested with a tampon mini-harvester (Skatron, Lier, Norway), heated at 65°C for 2 hours to inactivate virus, and counted in a Beckman 5500 gamma counter.

Data manipulation and statistics. Analyses were performed with Quattro 4.0 Spreadsheet program (Borland, Scotts Valley, Calif.) with an IBM AT-compatible computer. Percent specific lysis was calculated as the fraction [(experimental release-background release)/(Triton release-background release)]x100. The background was defined as the release in the absence of specific antibody, composed of spontaneous release (10-20%) and natural killing (0-20%), calculated by standard formulas [Junghans, R.P., Supra]. Standard deviations were typically 0-3% of input counts.

### Results

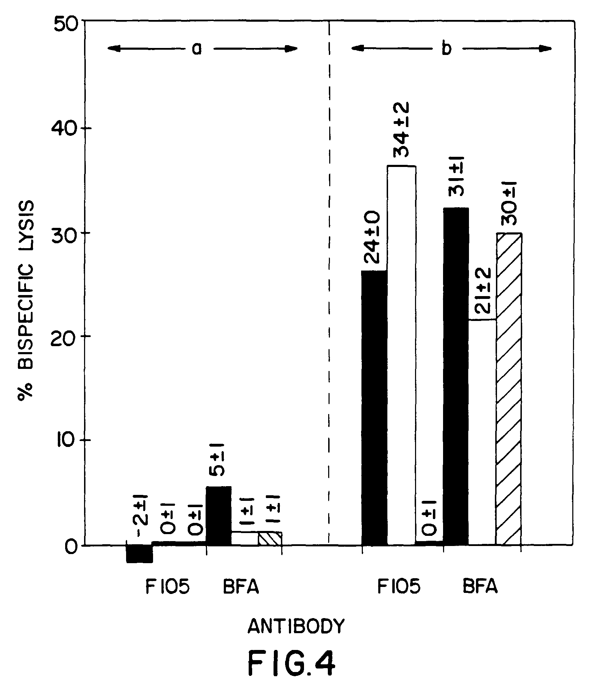

Specificity of bispecific immunoadhesin mediated cell lysis: Preliminary experiments were conducted to measure the (CD4 x anti-CD3) bispecific immunoadhesin mediated killing of HIV-1 infected and uninfected cells. HIV-1 IIIB infected and uninfected CEM and monocyte cell line U937 were used as targets. The results showed that HIV-1 infected CEM cells were lysed to a great extent with the bispecific immunoadhesin and with a control anti-gp120 antibody (F105) while uninfected cells showed no lysis (Fig. 4). The same results were observed with U937. Because the U937 cell line was much more sensitive to NK killing, causing higher backgrounds, it was not studied further (not shown). However, the pattern of killing with these antibodies differed with different effector cells. PBLs killed infected target cells in the presence of F105, or bispecific immunoadhesin, while CTL killed infected cells only in the presence of bispecific immunoadhesin and not with F105. In contrast to the bispecific immunoadhesin results of Fig. 4, PBLs mediated target cell lysis was generally enhanced when PBLs were stimulated with IL-2.

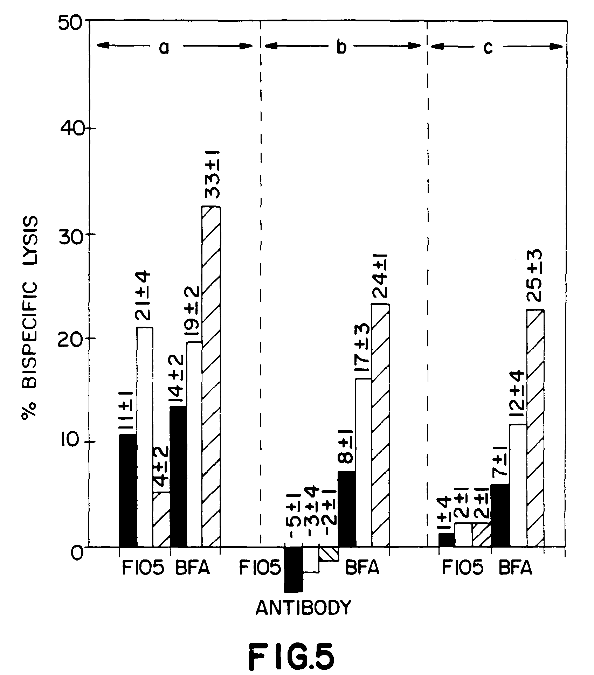

To mimic in vivo conditions, we tested the (CD4 x anti-CD3) bispecific immunoadhesin and F105 mediated target cell lysis in low and high concentrations of human serum (Fig. 5). F105 mediated lysis by PBL (+IL-2 or -IL-2) was completely blocked in 8% and 50% normal human serum, whereas the bispecific immunoadhesin mediated target cell lysis by PBL +IL-2 or -IL-2 and by CTL were only modestly affected.

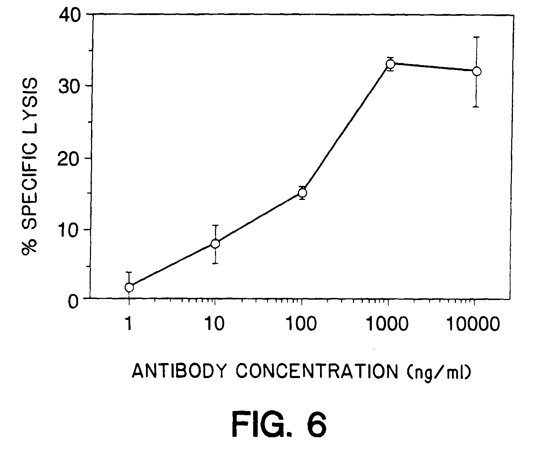

Bispecific immunoadhesin dose-dependent target cell lysis and its concentration curve. To test the most effective concentration of (CD4 x anti-CD3) bispecific immunoadhesin, we titrated the effects of different concentrations for mediating target cell lysis by PBL and CTL. As shown in Fig. 6, target cell lysis increased with higher bispecific immunoadhesin concentration until 1µg/ml, and slightly decreased or plateaued with higher concentrations. (CD4 x anti-CD3) bispecific immunoadhesin concentrations showed the same optimum in the presence of 50% human serum with PBL or CTL (not shown). Target cell killings by F105 also showed a plateau in the 1µg/ml range for PBL +IL-2 or -IL-2, and was not active in 50% human serum even at 10 µg/ml final concentration (not shown). These 1µg/ml concentrations were used for both antibodies in the studies of Figures 4 and 5.

### Discussion

The foregoing results confirm and extend previous reports that cytotoxic T lymphocytes are activated and retargeted by bifunctional antibodies to induce a dramatic lysis even in a short period (3 hours) of incubation.

Our data established killing through CTL recruitment in accord with the bifunctional immunoadhesin design. However, it was possible that target cell killing could also be mediated through recruitment of ADCC effectors. This could occur by bifunctional immunoadhesin binding to HIV antigen on the target cell surface through its CD4-IgG arm and binding to FCγ receptor of ADCC effectors through the bifunctional immunoadhesin Fc domain, as occurs with the parental CD4-IgG-1 (Byrn, R.A., *et al*., Supra) that comprises half of the bifunctional molecule. Contribution of ADCC to total activity in PBL appears to be small, however, as there is relatively modest effect of serum on cytotoxicity that is paralleled in the pure CTL preparation (which performs no ADCC), suggesting that this small reduction may be non-specific and ADCC-independent.

A merging of technologies for humanizing antibodies and preparing bifunctional constructs represents the latest in a series of responses designed to overcome problems of antibodies in therapy. With enhanced recruitment of host effector mechanisms demonstrated here and anticipated decreased immunogenicity and increased *in vivo* survivability, a still-hoped-for role for antibodies in therapy gains plausibility in treatments for infectious, immune and malignant diseases.

Although the foregoing refers to particular preferred embodiments, it will be understood that the present invention is not so limited. It will occur to those ordinarily skilled in the art that various modifications may be made to the disclosed embodiments without diverting from the overall concept of the invention. All such modifications are intended to be within the scope of the present invention.

## Claims

1. A method of making a bispecific immunoadhesin comprising:
a) introducing into a host cell DNA sequences encoding a first fusion comprising a first binding domain fused to an immunoglobulin heavy chain constant domain sequence lacking a light chain binding site; a second fusion comprising a second binding domain fused to an immunoglobulin heavy chain constant domain sequence retaining a light chain binding site; and an immunoglobulin light chain, respectively;
b) culturing said host cells so as to express said DNA sequences to produce a mixture of (i) a heterotrimer comprising said first fusion covalently linked with a second fusion-immunoglobulin light chain pair; (ii) a heterotetramer comprising two covalently-linked second fusion-immunoglobulin light chain pairs; and (iii) a homodimer comprising two covalently-linked molecules of said first fusion;
c) removing the mixture of products (i), (ii) and (iii) from the cell culture; and
d) isolating product (i) from products (ii) and (iii).

2. The method of claim 1 wherein product (i) is isolated in substantially homogenous form by separation methods based on the first binding domain and on the immunoglobulin light chain, respectively.

3. The method of claim 2 wherein products (i) and (iii) are separated from product (ii) by chromatography on an immunoaffinity column specific for the first binding domain; and product (i) is separated from product (iii) by chromatography on an immunoaffinity column specific for the immunoglobulin light chain.

4. The method of claim 2 wherein product (i) and (ii) are separated from product (iii) by chromatography on an immunoaffinity column specific for the immunoglobulin light chain; and product (i) is separated from product (ii) by chromatography on an immunoaffinity column specific for the first binding domain.

5. The method of claim 1 wherein the first fusion is devoid of the first immunoglobulin heavy chain constant domain (CH1), and the second fusion retains an intact CH1 domain.

6. The method of claim 5 wherein the first and second binding domains are selected from CD4 and L-selectin.

7. The method of claim 5 wherein the first binding domain comprises a CD4 amino acid sequence capable of gp 120 binding.

8. The method of claim 7 wherein the second binding domain comprises an anti-CD3 antibody antigen combining site.

9. The method of claim 8 wherein the DNA sequence encoding the first fusion is introduced into the host cell individually, in a vector capable of its expression.

10. The method of claim 9 wherein the DNA sequences encoding the second fusion and the immunoglobulin light chain are introduced into the host cell as part of the same vector capable of their expression.

11. The method of claim 9 wherein the DNA sequences encoding the second fusion and the immunoglobulin light chain are introduced into the host cell individually, in two distinct vectors capable of their expression.

12. The method of claim 11 wherein the weight percentage ratio of the DNA sequences encoding the first fusion, the second fusion and the immunoglobulin light chain is adjusted to about 70:20:10 - 60:20:20.

13. A method for making a substantially homogenous bispecific immunoadhesin with one arm specifically recognizing a foreign antigen on a target cell, and with the other arm an activation molecule on a cytotoxic cell, comprising:
a) introducing into host cells DNA sequences encoding a fusion of a foreign antigen binding domain with an immunoglobulin heavy chain constant domain sequence lacking a light chain binding site, a heavy chain of an antibody to an activation site on a cytotoxic cell, retaining a light chain binding site, and a light chain of said antibody;
b) culturing said host cells so as to express said DNA sequences to produce a mixture of (i) a heterotrimer bispecific immunoadhesin comprising a foreign antigen binding domain-immunoglobulin heavy chain fusion disulfide linked to a disulfide bonded antibody heavy chain-light chain pair; (ii) a disulfide-linked heterotetramer comprising two antibody heavy chain-light chain pairs; and (iii) a homodimer comprising two disulfide-linked foreign antigen binding domain-immunoglobulin heavy chain constant domain fusion;
c) removing the mixture of products (i), (ii) and (iii) from the cell culture;
d) separating the bispecific immunoadhesin (i) from products (ii) and (iii).

14. The method of claim 13 wherein the foreign antigen is an integral viral protein of human immunodeficiency virus (HIV).

15. The method of claim 14 wherein the foreign antigen binding domain is capable of specific binding to a site within the major envelope glycoprotein of HIV.

16. The method of claim 15 wherein the foreign antigen binding domain is a CD4 amino acid sequence.

17. The method of claim 16 wherein the cytotoxic cell is T cell lymphocyte or large granular lymphocyte.

18. The method of claim 17 wherein the activation molecule is CD3 or CD16.

19. The method of claim 18 wherein the antibody is an anti-CD3 antibody.

20. The method of claim 19 wherein the antibody is a humanized mouse anti-human CD3 antibody.

21. The method of claim 20 wherein the host cells are mammalian cells.

22. The method of claim 21 wherein the mammalian cells are cotransfected with three plasmids encoding and capable of expressing individually a CD4-IgG-1 immunoadhesin lacking the CH1 domain, a humanized mouse anti-human CD3 antibody heavy chain retaining a CH1 domain and a humanized mouse anti-human CD3 antibody light chain.

23. The method of claim 22 wherein the ratio of the three plasmids carrying the DNA sequences encoding the CD4-IgG-1 immunoadhesin lacking the CH1 domain, the humanized mouse anti-human CD3 antibody heavy chain retaining a CH1 domain and the humanized mouse anti-human CD3 antibody light chain, respectively is 60:20:20 percent.

24. Substantially homogenous bispecific immunoadhesin comprising a first binding domain fused to an immunoglobulin heavy chain constant domain sequence lacking an immunoglobulin light chain binding site covalently linked to the fusion of a second binding domain and an immunoglobulin heavy chain constant domain sequence retaining an immunoglobulin light chain binding site associated with an immunoglobulin light chain.

25. The bispecific immunoadhesin of claim 24 comprising a binding domain for an integral viral protein of a retrovirus fused to an immunoglobulin heavy chain constant domain sequence lacking an immunoglobulin light chain binding site covalently linked to a heavy chain-light chain pair of an antibody to an activation molecule on the surface of a cytotoxic cell.

26. The bispecific immunoadhesin of claim 25 wherein the integral viral protein is the gp 120 domain of the major envelope glycoprotein of HIV.

27. The bispecific immunoadhesin of claim 26 wherein the gp 120 binding domain is a CD4 amino acid sequence.

28. The bispecific immunoadhesin of claim 27 wherein the first two variable domains of a CD4 amino acid sequence are fused to hinge, CH2 and CH3 amino acid sequences from an IgG immunoglobulin.

29. The bispecific immunoadhesin of claim 28 essentially as shown in Figure 1.

30. A trimeric bispecific immunoadhesin with one arm specifically recognizing HIV on a target cell, and with the other arm an activation molecule on a cytotoxic cell, for use in the prevention or treatment of HIV infection in a human exposed to HIV.

31. A trimeric bispecific IgG immunoadhesin with one arm specifically recognizing HIV on a target cell, and with the other arm an activation molecule on a cytotoxic cell for use in preventing the transmission of HIV infection from an HIV seropositive pregnant woman to the fetus.

## Patentansprüche

1. Verfahren zur Herstellung eines bispezifischen Immunadhesins, umfassend:
a) das Einbringen von DNA-Sequenzen, die für folgendes kodieren: eine erste Fusion, umfassend eine erste Bindedomäne, die mit einer Konstantdomänen-Sequenz einer schweren Kette eines Immunglobulins fusioniert ist, welche Sequenz keine Bindestelle für eine leichte Kette aufweist; eine zweite Fusion, umfassend eine zweite Bindedomäne, die mit einer Konstantdomänen-Sequenz einer schweren Kette eines Immunglobulins fusioniert ist, welche Sequenz eine Leichtketten-Bindestelle behält; bzw. eine leichte Kette eines Immunglobulins, in eine Wirtzelle;
b) das Kultivieren der Wirtzellen, um die DNA-Sequenzen zu exprimieren, sodaß ein Gemisch aus (i) einem Heterotrimer, das die erste Fusion umfaßt, die kovalent mit dem aus der zweiten Fusion und der leichten Kette des Immunglobulins bestehenden Paar verbunden ist; (ii) einem Heterotetramer, das zwei kovalent verbundene, aus der zweiten Fusion und der leichten Kette eines Immunglobulins bestehende Paare umfaßt; und (iii) einem Homodimer, das zwei kovalent verbundene Moleküle der ersten Fusion umfaßt, gebildet wird;
c) das Entfernen des Gemisches der Produkte (i), (ii) und (iii) aus der Zellkultur; und
(d) das Isolieren des Produkts (i) von den Produkten (ii) und (iii) .

2. Verfahren nach Anspruch 1, worin Produkt (i) in im wesentlichen homogener Form durch Trennungsverfahren auf der Basis der ersten Bindedomäne bzw. der leichten Kette des Immunglobulins isoliert wird.

3. Verfahren nach Anspruch 2, worin die Produkte (i) und (iii) von Produkt (ii) durch Chromatografie auf einer Immunaffinitätssäule getrennt werden, die für die erste Bindedomäne spezifisch ist; und Produkt (i) von Produkt (iii) durch Chromatografie auf einer Immunaffinitätssäule getrennt wird, die für die leichte Kette des Immunglobulins spezifisch ist.

4. Verfahren nach Anspruch 2, worin Produkt (i) und (ii) von Produkt (iii) durch Chromatografie auf einer Immunaffinitätssäule getrennt werden, die für die leichte Kette des Immunglobulins spezifisch ist; und Produkt (i) von Produkt (ii) durch Chromatografie auf einer Immunaffinitätssäule getrennt wird, die für die erste Bindedomäne spezifisch ist.

5. Verfahren nach Anspruch 1, worin die erste Fusion die erste Konstantdomäne der schweren Kette des Immunglobulins (CH1) nicht aufweist und die zweite Fusion eine intakte CH1-Domäne behält.

6. Verfahren nach Anspruch 5, worin die erste und zweite Bindedomäne aus CD4 und L-Selectin ausgewählt sind.

7. Verfahren nach Anspruch 5, worin die erste Bindedomäne eine CD4-Aminosäuresequenz umfaßt, die gp120 binden kann.

8. Verfahren nach Anspruch 7, worin die zweite Bindedomäne eine anti-CD3-Antikörper-Antigen-Kombinationsstelle umfaßt.

9. Verfahren nach Anspruch 8, worin die für die erste Fusion kodierende DNA-Sequenz einzeln, in einem zu ihrer Expression fähigen Vektor, in die Wirtszelle eingesetzt wird.

10. Verfahren nach Anspruch 9, worin die für die zweite Fusion und die leichte Kette des Immunglobulins kodierenden DNA-Sequenzen als Teil des gleichen, zu ihrer Expression fähigen Vektors in die Wirtzelle eingesetzt werden.

11. Verfahren nach Anspruch 9, worin die für die zweite Fusion und die leichte Kette des Immunglobulins kodierenden DNA-Sequenzen einzeln, in zwei unterschiedlichen, zu ihrer Expression fähigen Vektoren, in die Wirtzelle eingesetzt werden.

12. Verfahren nach Anspruch 11, worin das Gew.-%-Verhältnis der für die erste Fusion, die zweite Fusion und die leichte Kette des Immunglobulins kodierenden DNA-Sequenzen auf etwa 70:20:10 - 60:20:20 eingestellt wird.

13. Verfahren zur Herstellung eines im wesentlichen homogenen bispezifischen Immunadhesins, dessen ein Arm ein Fremdantigen auf einer Zielzelle und dessen anderer Arm ein Aktivierungsmolekül auf einer zytotoxischen Zelle spezifisch erkennt, umfassend:
a) das Einbringen von DNA-Sequenzen in Wirtzellen, wobei die Sequenzen für eine Fusion einer Fremdantigen-Bindedomäne mit einer Konstantdomänen-Sequenz einer schweren Kette eines Immunglobulins, wobei diese Sequenz keine Bindestelle für eine leichte Kette aufweist, für eine schwere Kette eines Antikörpers gegen eine Aktivierungssteile auf einer zytotoxischen Zelle, wobei eine Bindestelle für eine leichte Kette erhalten bleibt, und für eine leichte Kette dieses Antikörpers kodieren;
b) das Kultivieren der Wirtzellen, um die DNA-Sequenzen zu exprimieren, sodaß ein Gemisch aus (i) einem heterotrimeren bispezifischen Immunadhesin, umfassend eine Fusion einer Fremdantigen-Bindedomäne mit der schweren Kette eines Immunglobulins, die über eine Disulfidbrücke mit einem durch Disulfidbindung verbundenen Paar, bestehend aus einer leichten Kette und einer schweren Kette eines Antikörpers, verbunden ist; aus (ii) einem disulfidverbundenen Heterotetramer mit zwei Paaren, bestehend aus einer leichten Kette und einer schweren Kette eines Antikörpers; und aus (iii) einem Homodimer, das zwei über eine Disulfidbrücke verbundene Fusionen einer Fremdantigen-Bindedomäne mit der Konstantdomäne einer schweren Kette eines Immunglobulins umfaßt, hergestellt wird;
c) das Entfernen des Gemisches der Produkte (i), (ii) und (iii) aus der Zellkultur;
d) das Trennen des bispezifischen Immunadhesins (i) von Produkten (ii) und (iii).

14. Verfahren nach Anspruch 13, worin das Fremdantigen ein integrales virales Protein des menschlichen Immunschwächevirus (HIV) ist.

15. Verfahren nach Anspruch 14, worin die Fremdantigen-Bindedomäne zur spezifischen Bindung an eine Stelle im Haupt-Hüllenglykoprotein von HIV fähig ist.

16. Verfahren nach Anspruch 15, worin die Fremdantigen-Bindedomäne eine CD4-Aminosäuresequenz ist.

17. Verfahren nach Anspruch 16, worin die zytotoxische Zelle ein T-Zellenlymphozyt oder ein großer granulärer Lymphozyt ist.

18. Verfahren nach Anspruch 17, worin das Aktivierungsmolekül CD3 oder CD16 ist.

19. Verfahren nach Anspruch 18, worin der Antikörper ein anti-CD3-Antikörper ist.

20. Verfahren nach Anspruch 19, worin der Antikörper ein humanisierter anti-menschlicher CD3-Antikörper aus Mäusen ist.

21. Verfahren nach Anspruch 20, worin die Wirtzellen Säugetierzellen sind.

22. Verfahren nach Anspruch 21, worin die Säugetierzellen mit drei Plasmiden co-transfiziert werden, die einzeln für ein CD4-lgG-1-Immunadhesin ohne CH1-Domäne, eine schwere Kette eines humanisierten anti-menschlicher CD3-Antikörpers aus Mäusen mit einer CH1-Domäne und eine leichte Kette eines humanisierten anti-menschlicher CD3-Antikörpers kodieren und diese einzeln exprimieren können.

23. Verfahren nach Anspruch 22, worin das Verhältnis der drei Plasmide, die die DNA-Sequenzen tragen, die für das CD4-lgG-1-Immunadhesin ohne CH1-Domäne, eine schwere Kette eines humanisierten anti-menschlicher CD3-Antikörpers aus Mäusen mit einer CH1-Domäne bzw. eine leichte Kette eines humanisierten anti-menschlicher CD3-Antikörpers kodieren, 60:20:20 % beträgt.

24. Im wesentlichen homogenes bispezifisches Immunadhesin, umfassend eine erste Bindedomäne, die mit einer Konstantdomänensequenz einer schweren Kette eines Immunglobulins, der eine Bindestelle für eine leichte Kette eines Immunglobulins fehlt, fusioniert ist, und die kovalent mit der Fusion einer zweiten Bindedomäne mit einer Konstantdomänensequenz einer schweren Kette eines Immunglobulins verbunden ist, die eine mit einer leichten Kette eines Immunglobulins assoziierte Bindestelle für eine leichte Kette eines Immunglobulins aufweist.

25. Bispezifisches Immunadhesin nach Anspruch 24, umfassend eine Bindedomäne für ein integrales virales Protein eines Retrovirus, die mit einer Konstantdomänensequenz einer schweren Kette eines Immunglobulins, der eine Bindestelle für eine leichte Kette eines Immunglobulins fehlt, fusioniert ist, und die kovalent mit einem aus den leichten und schweren Ketten eines Antikörpers gegen ein Aktivierungsmolekül auf der Oberfläche einer zytotoxischen Zelle bestehenden Paar verbunden ist.

26. Bispezifisches Immunadhesin nach Anspruch 25, worin das integrale virale Protein die gp120-Domäne des Haupt-Hüllenglykoproteins von HIV ist.

27. Bispezifisches Immunadhesin nach Anspruch 26, worin die gp120-Bindedomäne eine CD4-Aminosäuresequenz ist.

28. Bispezifisches Immunadhesin nach Anspruch 27, worin die ersten zwei variablen Domänen einer CD4-Aminosäuresequenz mit Hinge- ("Scharnier"-), CH2- und CH3-Aminosäuresequenzen aus einem IgG-Immunglobulin fusioniert sind.

29. Bispezifisches Immunadhesin nach Anspruch 28, das im wesentlichen so wie in Fig.1 ist.

30. Trimeres bispezifisches Immunadhesin mit einem Arm, der spezifisch HIV auf einer Zielzelle und einem anderen Arm, der ein Aktivierungsmolekül auf einer zytotoxischen Zelle erkennt, zur Verwendung bei der Prävention oder Behandlung von HIV-Infektion in einem Menschen, der HIV ausgesetzt ist.

31. Trimeres bispezifisches IgG-Immunadhesin mit einem Arm, der spezifisch HIV auf einer Zielzelle, und einem anderen Arm, der ein Aktivierungsmolekül auf einer zytotoxischen Zelle erkennt, zur Verwendung bei der Verhinderung der Übertragung der HIV-Infektion von einer HIV-seropositiven schwangeren Frau auf den Fötus.

## Revendications

1. Procédé de préparation d'une immunoadhésine bispécifique, comprenant :
a) l'introduction dans une cellule-hôte de séquences d'ADN codant pour une première fusion comprenant un premier domaine de liaison fusionné à une séquence de domaine constant de chaîne lourde d'immunoglobuline dépourvue d'un site de liaison de chaîne légère ; une seconde fusion comprenant un second domaine de liaison fusionné à une séquence de domaine constant de chaîne lourde d'immunoglobuline conservant un site de liaison de chaîne légère ; et une chaîne légère d'immunoglobuline, respectivement ;
b) la culture desdites cellules-hôtes de manière à exprimer lesdites séquences d'ADN pour produire un mélange (i) d'un hétérotrimère comprenant ladite première fusion liée par covalence à une paire seconde fusion-chaîne légère d'immunoglobuline ; (ii) d'un hétérotétramère comprenant deux paires seconde fusion-chaîne légère d'immunoglobuline liées par covalence ; et (iii) d'un homodimère comprenant deux molécules liées par covalence de ladite première fusion ;
c) la séparation du mélange de produits (i), (ii) et (iii) de la culture de cellules ; et
d) la séparation du produit (i) des produits (ii) et (iii).

2. Procédé suivant la revendication 1, dans lequel le produit (i) est isolé sous une forme pratiquement homogène par des procédés de séparation basés sur le premier domaine de liaison et sur la chaîne légère d'immunoglobuline, respectivement.

3. Procédé suivant la revendication 2, dans lequel les produits (i) et (iii) sont séparés du produit (ii) par chromatographie sur une colonne d'immuno-affinité spécifique du premier domaine de liaison ; et le produit (i) est séparé du produit (iii) par chromatographie sur une colonne d'immuno-affinité spécifique de la chaîne légère d'immunoglobuline.

4. Procédé suivant la revendication 2, dans lequel les produits (i) et (ii) sont séparés du produit (iii) par chromatographie sur une colonne d'immuno-affinité spécifique de la chaîne légère d'immunoglobuline ; et le produit (i) est séparé du produit (ii) par chromatographie sur une colonne d'immuno-affinité spécifique du premier domaine de liaison.

5. Procédé suivant la revendication 1, dans lequel la première fusion est dépourvue du premier domaine constant de chaîne légère d'immunoglobuline (CH1), et la seconde fusion conserve un domaine CH1 intact.

6. Procédé suivant la revendication 5, dans lequel les premier et second domaines de liaison sont choisis entre CD4 et la L-sélectine.

7. Procédé suivant la revendication 5, dans lequel le premier domaine de liaison comprend une séquence d'aminoacides de CD4 apte à la liaison avec GP 120.

8. Procédé suivant la revendication 7, dans lequel le second domaine de liaison comprend un site de combinaison d'antigène et d'anticorps anti-CD3.

9. Procédé suivant la revendication 8, dans lequel la séquence d'ADN codant pour la première fusion est introduite dans la cellule hôte individuellement, dans un vecteur apte à son expression.

10. Procédé suivant la revendication 9, dans lequel la séquence d'ADN codant pour la seconde fusion et la chaîne légère d'immunoglobuline sont introduites dans la cellule hôte en tant qu'une partie du même vecteur apte à leur expression.

11. Procédé suivant la revendication 9, dans lequel les séquences d'ADN codant pour la seconde fusion et la chaîne légère d'immunoglobuline sont introduites dans la cellule hôte individuellement, dans deux vecteurs distincts aptes à leur expression.

12. Procédé suivant la revendicationo 11, dans lequel le rapport, en pourcentage pondéral, des séquences d'ADN codant pour la première fusion, la seconde fusion et la chaîne légère d'immunoglobuline est ajusté à une valeur d'environ 70:20:10 à 60:20:20.

13. Procédé pour la préparation d'une immunoadhésine bispécifique essentiellement homogène comportant un bras reconnaissant spécifiquement un antigène étranger sur une cellule cible, et l'autre bras reconnaissant spécifiquement une molécule d'activation sur une cellule cytotoxique, comprenant ;
a) l'introduction dans des cellules hôtes de séquences d'ADN codant pour une fusion d'un domaine de liaison d'antigène étranger avec une séquence de domaine constant de chaîne lourde d'immunoglobuline dépourvue de site de liaison de chaîne légère, d'une chaîne lourde d'un anticorps contre un site d'activation sur une cellule cytotoxique, conservant un site de liaison de chaîne légère, et d'une chaîne légère dudit anticorps ;
b) la culture desdites cellules hôtes de manière à exprimer lesdites séquences d'ADN pour produire un mélange (i) d'une immunoadhésine bispécifique hétérotrimère comprenant une fusion domaine de liaison d'antigène étranger-chaîne lourde d'immunoglobuline liée par des ponts disulfure à une paire chaîne légère-chaîne lourde d'anticorps liés par des ponts disulfure ; (ii) d'un hétérotétramère lié par des ponts disulfure, comprenant deux paires chaîne légère-chaîne lourde d'anticorps ; et (iii) d'un homodimère comprenant deux fusions domaine de liaison d'antigène étranger-domaine constant de chaîne lourde d'immunoglobuline liés par des ponts disulfure ;
c) la séparation du mélange de produits (i), (ii) et (iii) de la culture de cellules ;
d) la séparation de l'immunoadhésine bispécifique des produits (ii) et (iii).

14. Procédé suivant la revendication 13, dans lequel l'antigène étranger est une protéine virale intégrale du virus d'immunodéficience humaine (HIV).

15. Procédé suivant la revendication 14, dans lequel le domaine de liaison d'antigène étranger est apte à la liaison spécifique à un site à l'intérieur de la glycoprotéine majeure d'enveloppe de HIV.

16. Procédé suivant la revendication 15, dans lequel le domaine de liaison d'antigène étranger est une séquence d'aminoacides de CD4.

17. Procédé suivant la revendication 16, dans lequel la cellule cytotoxique est un lymphocyte T ou un grand lymphocyte granulaire.

18. Procédé suivant la revendication 17, dans lequel la molécule d'activation est une molécule CD3 ou CD16.

19. Procédé suivant la revendication 18, dans lequel l'anticorps est un anticorps anti-CD3.

20. Procédé suivant la revendication 19, dans lequel l'anticorps est un anticorps de souris anti-CD3 humaine, adapté à l'homme.

21. Procédé suivant la revendication 20, dans lequel les cellules hôtes sont des cellules de mammifère.

22. Procédé suivant la revendication 21, dans lequel les cellules de mammifère sont cotransfectées avec trois plasmides codants pour, et aptes à l'expression individuellement de, une immunoadhésine CD4 IgG-1 dépourvue du domaine CH1, une chaîne lourde d'anticorps de souris anti-CD3 humaine adapté à l'homme conservant un domaine CH1 et une chaîne légère d'anticorps de souris anti-CD3 humaine adapté à l'homme.

23. Procédé suivant la revendication 22, dans lequel le rapport des trois plasmides portant les séquences d'ADN codants pour l'immunoadhésine CD4 IgG-1 dépourvue du domaine CH1, la chaîne lourde d'anticorps de souris anti-CD3 humaine adapté à l'homme conservant un domaine CH1 et la chaîne légère d'anticorps de souris anti-CD3 humaine adapté à l'homme, respectivement, est égale à 60:20:20 pour cent.

24. Immunoadhésine bispécifique essentiellement homogène comprenant un premier domaine de liaison fusionné à une séquence de domaine constant à chaîne lourde d'immunoglobuline dépourvue de site de liaison de chaîne légère d'immunoglobuline, liée par covalence à la fusion d'un second domaine de liaison et d'une séquence de domaine constant de chaîne lourde d'immunoglobuline conservant un site de liaison de chaîne légère d'immunoglobuline associée à une chaîne légère d'immunoglobuline.

25. Immunoadhésine bispécifique suivant la revendication 24, comprenant un domaine de liaison pour une protéine virale intégrale d'un rétrovirus fusionné à une séquence de domaine constant de chaîne d'immunoglobuline dépourvue de site de liaison de chaîne légère d'immunoglobuline, liée par covalence à une paire chaîne lourde-chaîne légère d'un anticorps contre une molécule d'activation sur la surface d'une cellule cytotoxique.

26. Immunoadhésine bispécifique suivant la revendication 25, dans laquelle la protéine virale intégrale est le domaine gp 120 de la glycoprotéine majeure d'enveloppe de HIV.

27. Immunoadhésine bispécifique suivant la revendication 26, dans laquelle le domaine de liaison gp 120 est une séquence d'aminoacides de CD4.

28. Immunoadhésine bispécifique suivant la revendication 27, dans laquelle les deux premiers domaines variables d'une séquence d'aminoacides de CD4 sont fusionnés à des séquences d'aminoacides de segment intermédiaire, CH2 et CH3 d'une immunogluline IgG.

29. Immunoadhésine bispécifique suivant la revendication 28, essentiellement de la manière représentée sur la figure 1.

30. Immunoadhésine bispécifique trimère, comportant un bras reconnaissant spécifiquement le HIV sur une cellule cible, et l'autre bras reconnaissant spécifiquement une molécule d'activation sur une cellule cytotoxique, destinée à être utilisée dans la prévention ou le traitement d'une infection par le HIV chez un sujet humain exposé au HIV.

31. Immunoadhésine IgG bispécifique trimère, comportant un bras reconnaissant spécifiquement le HIV sur une cellule cible, et l'autre bras reconnaissant spécifiquement une molécule d'activation sur une cellule cytotoxique, destinée à être utilisée dans la prévention de la transmission d'une infection par le HIV d'une femme enceinte HIV-séropositive au foetus.
